(19) Europäisches Patentamt
European Patent Office
Office européen des brevets

(11) Publication number: **0 003 666**
**B1**

(12) **EUROPEAN PATENT SPECIFICATION**

(45) Date of publication of patent specification: **06.04.83**

(21) Application number: **79300173.6**

(22) Date of filing: **02.02.79**

(51) Int. Cl.³: **C 07 D 307/93,**
C 07 C 61/04,
C 07 C 67/00,
C 07 C 69/38,
C 07 C 69/72,
C 07 C 69/74,
C 07 C 113/00 //A01N53/00

(54) **Oxabicyclo 3.1.0 hexanes useful in the production of cyclopropanecarboxylate insecticides and intermediate compounds.**

(30) Priority: **06.02.78 US 875648**
**06.02.78 US 875649**
**03.01.79 US 736**

(43) Date of publication of application:
**22.08.79 Bulletin 79/17**

(45) Publication of the grant of the patent:
**06.04.83 Bulletin 83/14**

(84) Designated Contracting States:
**BE CH DE FR GB LU NL**

(56) References cited:
DD - A - 84 186
DE - A - 2 649 856
GB - A - 1 283 225

**CHEMICAL ABSTRACTS volumes 66—75, eight collective index, formulas, page 2571 F**

(73) Proprietor: **FMC Corporation**
**2000 Market Street**
**Philadelphia Pennsylvania 19103 (US)**

(72) Inventor: **Hatch, Charles Eldrige**
**284 Wargo Road**
**Pennington New Jersey 08534 (US)**
Inventor: **Kondo, Kiyoshi**
**Chuo-Rinkan 5-16-4**
**Yamato Kanagawa (JP)**
Inventor: **Takashima, Toshiyuki**
**Kokubinjidai 1-7-20**
**Ebina Kanagawa (JP)**
Inventor: **Tunemoto, Daiei**
**Minamidai-1-9-2**
**Sagamihara Kanagawa (JP)**

(74) Representative: **Crampton, Keith John Allen et al,**
**D YOUNG & CO 10 Staple Inn**
**London WC1V 7RD (GB)**

(56) References cited:
**CHEMICAL ABSTRACTS vol. 70, no. 15, 14 April 1969, Columbus, Ohio, USA,**
**G. CANNIC et al.: "Cyclization of the carbenes obtained from the decomposition of allyl ethyl diazomalonates or allyl diazoacetoacetates" page 305, abstract no. 67699y**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 003 666 B1**

Courier Press, Leamington Spa, England

Oxabicyclo[3.1.0]hexanes useful in the production of cyclopropanecarboxylate insecticides and intermediate compounds

This invention concerns intermediates for the production of *cis*-3-(2,2-dihalovinyl)-2,2-dimethylcyclopropanecarboxylates, which are pyrethroid insecticides, and processes for making the intermediates.

Pyrethrins, naturally-occurring extracts of chrysanthemum flowers, have long been of interest as insecticides. Certain man-made variations of the natural pyrethrins are much more potent than the natural materials and exhibit other advantages. The prior art discloses 3-(2,2-dihalovinyl)-2,2-dimethylcyclopropanecarboxylic acids, corresponding lower alkyl esters, and pyrethroid insecticides prepared from them. The acids and esters contain the following carboxylate radical, wherein X is chlorine or bromine.

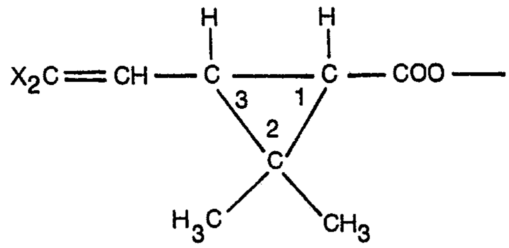

To produce pyrethroid insecticides, the acids or the lower alkyl esters may be esterified (or transesterified) with various alcohols, for example, m-phenoxybenzyl alcohol, $\alpha$-cyano-m-phenoxybenzyl alcohol, and other alcohols well known to those skilled in the art.

These acids and esters are known to exist as both *cis* and *trans* geometrical isomers; the carboxy and dihalovinyl groups at C—1 and C—3 may be either *cis* or *trans* with respect to each other. There are substantial differences in insecticidal activity between pyrethroid esters made from the *cis* and *trans* isomers of a given dihalovinylcyclopropanecarboxylic acid. In general, as between the *cis* and *trans* isomers of a given synthetic pyrethroid ester, the *cis* isomer is more active than the *trans*; the relative activities are reversed in the natural esters.

C—1 and C—3 are asymmetric, and the pyrethroid esters may be optically active, their activity depending upon the stereoconfigurations at C—1 and C—3. There are four optical isomers, (1R,3R), (1S,3R), (1R,3S), and (1S,3S). The (1R,3R) and (1S,3S) isomers are *cis*, whereas the (1S,3R) and (1R,3S) isomers are *trans*. In general, the (1R,3R) isomers are the most active.

Because of the greater insecticidal activity of the *cis* esters, processes for producing *cis*-3-(2,2-dihalovinyl)-2,2-dimethylcyclopropanecarboxylates, essentially free of the *trans* isomers, are actively being sought, and between the two optical isomers comprising a racemic *cis* geometrical isomer, the (1R,3R) optical isomer is especially desired. For example, 3-phenoxybenzyl (1R,3R)-3-(2,2-dichlorovinyl)-2,2-dimethylcyclopropanecarboxylate and (S)-$\alpha$-cyano-3-phenoxybenzyl (1R,3R)-3-(2,2-dibromovinyl)-2,2-dimethylcyclopropanecarboxylate are especially active, more active than the (1S,3S) isomers, and are of great commercial interest.

The *cis*-3-(2,2-dihalovinyl)-2,2-dimethylcyclopropanecarboxylic acids and lower alkyl esters produced according to this invention may be esterified and converted into pyrethroid insecticides, with essentially no racemization or isomerization, according to methods disclosed in the prior art and well known to those skilled in the art. For example, the acid may be treated with thionyl chloride to produce the acid chloride, followed by reaction with an appropriate alcohol to produce an insecticidal ester, and the lower alkyl ester may be transesterified with an appropriate alcohol.

This invention includes both processes and compounds. In one process, this invention provides a process for preparing a lactone of the formula

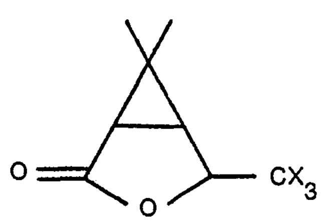

where each X is a chlorine or bromine atom, which comprises cyclizing a diazo ester of the formula

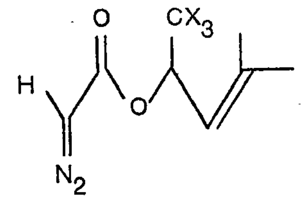

by intramolecular carbenoid cyclization. The process may be carried out stereospecifically.

In other aspects, this invention provides processes, which may be carried out stereospecifically in some instances, for the production of novel intermediates, for example, malonic and alkanoylacetic esters of 3-methyl-2-buten-2-ol or a 1,1,1-trihalo-4-methyl-3-penten-2-ol, as well as diazo derivatives of the esters, and 1-alkoxycarbonyl-substituted lactones, all useful as starting materials for the production of lactones represented by the aforesaid formula.

In yet another aspect, this invention provides a process for preparing, essentially free of the *trans* isomer, a *cis*-3-(2,2-dihalovinyl)-2,2-dimethylcyclopropanecarboxylic acid of the formula

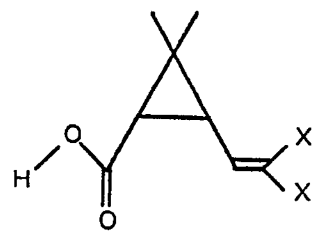

wherein the X's are the same or different, each X is a chlorine or bromine atom, and the carboxy and dihalovinyl groups are *cis* with respect to each other, which comprises subjecting to an elimination reaction, for example, treating with zinc and acetic acid, a compound of the formula

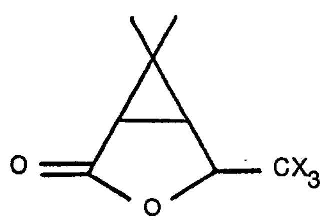

wherein the X's are the same or different and have the values given above, thereby simultaneously opening the lactone ring and eliminating one X atom. This process too may produce an optically active product.

This invention also provides novel intermediate compounds useful for the production of *cis*-3-(2,2-dihalovinyl)-2,2-dimethylcyclopropanecarboxylic acids and lower alkyl esters, from which pyrethroid insecticides may be prepared as described above.

The compounds of the invention can be represented collectively by structural formulae I, II and III below, where the X's are the same or different, each X is a chlorine or bromine atom, $R^1$ is lower alkyl or lower alkoxy, $R^2$ is hydrogen, lower alkoxycarbonyl, or lower alkanoyl, and $R^3$ is lower alkoxycarbonyl. The term "lower" as used herein to modify expressions such as alkyl, alkoxy, and so forth, means a chain of 1—6, preferably 1—4 carbon atoms.

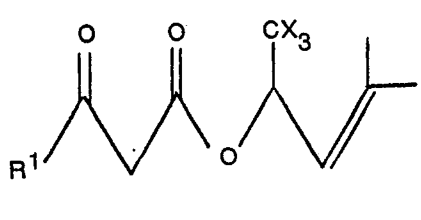

I

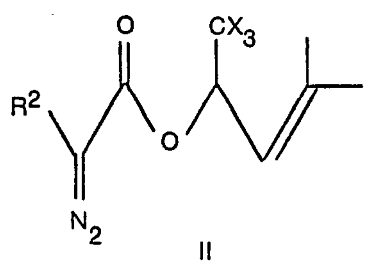

II

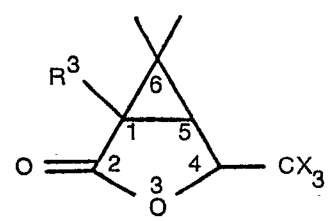

III

The compounds of formula I are esters of alkanoylacetic and malonic acids, that is, $R^1$ is lower alkyl or lower alkoxy, respectively. These compounds are chiral. These novel esters may be prepared by esterifying 1,1,1-trihalo-4-methyl-3-penten-2-ols; for example, with diketene or a lower alkylmalonyl chloride.

The compounds of formula II, that is, the diazo derivatives of the aforesaid esters, which are chiral, and the lactones of formula III, which are also chiral, can be produced from the esters of formula I by process embodiments of this invention, as can the *cis*-3-(2,2-dihalovinyl)-2,2-dimethylcyclopropane carboxylic acids and lower alkyl esters — all as described hereinafter. It should further be noted that United Kingdom Patent No. 1 283 225 discloses certain 3-alkoxyoxabicyclo[3.1.0]hexanes, which have the same basic ring as Compound III.

An alkanoyl group $R^2$ and an alkoxycarbonyl group $R^3$ (values arising when $R^1$ is lower alkyl or lower alkoxy, respectively, in the ester precursors of Formula I) can be cleaved from the respective derivatives of Formulae II and III.

Processes and compounds of this invention are exemplified in the following diagram, in which R is H or $CX_3$ and the other variables are as already defined. The invention is not to be construed as limited to the specific embodiments illustrated, which are described in detail after the diagram.

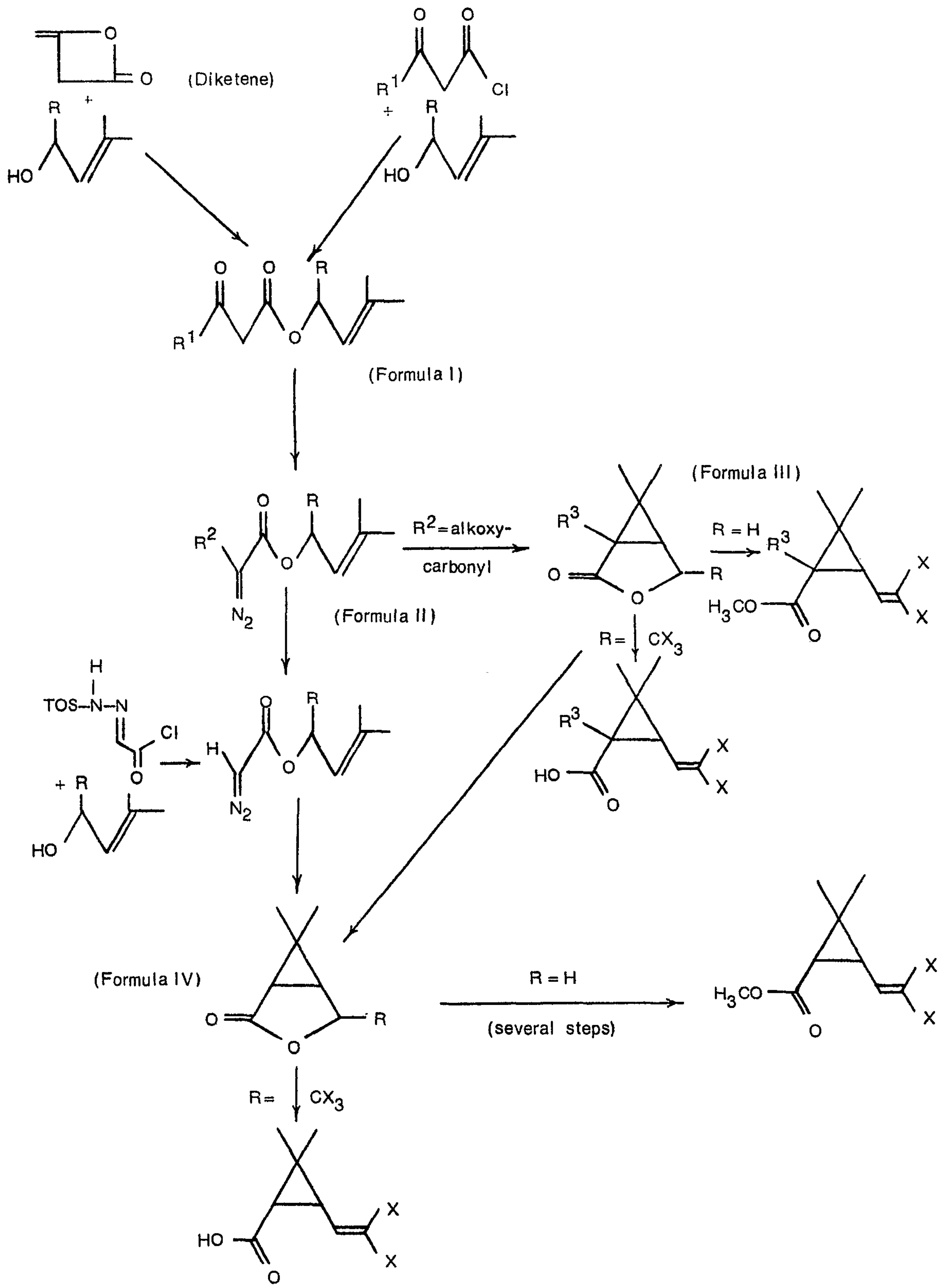


In one process, an ester of formula I is subjected to a diazo transfer reaction, for example, by reacting it with an azide, such as an arenesulfonyl azide, for example, tosyl azide or "polymer-bound" sulfonyl azide, to produce the corresponding diazo compound of formula II.

The ester of formula I is chiral, and when the ester is the (R) isomer, the diazo compound of formula II is also the (R) isomer. In the following description of the process, where reference is made to an (R) optical isomer it will be understood that an equivalent statement can be made for the

corresponding compound in the (S) series, and conversely.

When the ester of formula I is a 3-methyl-1-trihalomethyl-2-butenyl alkanoylacetate ($R^1$ is lower alkyl), a 3-methyl-1-trihalomethyl-2-butenyl 2-diazoalkanoylacetate (formula II, $R^2$ is lower alkanoyl), is produced. Preferably, without isolation of this intermediate, the alkanoyl group is cleaved, for example, with base, preferably aqueous sodium hydroxide, to yield directly a 3-methyl-1-trihalomethyl-2-butenyl diazoacetate (formula II, $R^2$ is hydrogen), respectively. 3-Methyl-1-trihalomethyl-2-butenyl acetoacetates are especially preferred as the ester of formula I, since they may be prepared readily from diketene and 1,1,1-trihalo-4-methyl-3-penten-2-ols. Accordingly, (R)-3-methyl-1-trichloromethyl-2-butenyl diazoacetate is produced from (R)-3-methyl-1-trichloromethyl-2-butenyl acetoacetate.

When the ester of formula I is a 3-methyl-1-trihalomethyl-2-butenyl malonate ($R^1$ is lower alkoxy), it is preferred that the ethyl ester be used in the diazo transfer reaction, producing an ethyl 3-methyl-1-trihalomethyl-2-butenyl diazomalonate (formula II, $R^2$ is ethoxycarbonyl). In this manner, ethyl (R)-3-methyl-1-trichloromethyl-2-butenyl diazomalonate is produced from ethyl (R)-3-methyl-1-trichloromethyl-2-butenyl malonate.

It is preferable, however, to prepared a diazoacetate, rather than a diazomalonate, since the diazoacetate contains the hydrogen atom ultimately desired at C—1 of the *cis*-3-(2,2-dihalovinyl)-2,2-dimethylcyclopropanecarboxylic acid or ester. Thus, it is preferred to use a 3-methyl-1-trihalomethyl-2-butenyl alkanoylacetate (formula I, $R^1$ is lower alkyl, for example, methyl) as the starting material, and an acetoacetate is especially preferred.

A second process, also within the scope of this invention, for producing the preferred 3-methyl-1-trihalomethyl-2-butenyl diazoacetate is to esterify a 1,1,1-trihalo-4-methyl-3-penten-2-ol with a hydrazone, for example, the tosyl hydrazone, of glyoxyloyl chloride.

In a third process, a diazo derivative of formula II (compounds of formula II are chiral), in which $R^2$ is hydrogen or lower alkoxycarbonyl, which may be prepared as described above, is cyclized by intramolecular carbenoid cyclization, for example, by treatment with a copper-containing catalyst, thereby yielding a bicyclic lactone, that is, a 4-trihalomethyl-6,6-dimethyl-2-oxo-3-oxabicyclo[3.1.0]hexane of formula III. Preferably, the starting compound is a diazoacetate, thereby producing a compound of formula III in which $R^3$ is hydrogen.

If the copper-containing catalyst is not optically active, a racemic lactone is obtained, unless the diazo derivative of formula II is optically active. In that case, the (R) isomer of the diazo derivative yields a (1R,4R,5S)-4-trihalomethyl-6,6-dimethyl-2-oxo-3-oxabicyclo[3.1.0]hexane, substantially free of other isomers thereof. A diazo derivative of (S) stereochemistry produces a (1S,4S,5R) lactone. The compounds of formula III are capable of existing as four optical isomers, and it is particularly surprising that a single optical isomer is obtained, since two new asymmetric carbon atoms, C—1 and C—5 in the lactone, are produced. Molecular models of the diazo ester precursors of formula II suggest that a carbenoid intermediate produced by the elimination of nitrogen from the compound of formula II would cyclize by attack at either side of the double bond with approximately equal probability. In reality, attack occurs selectively at only one face of the olefin.

The cyclization also affords an optically active lactone when chiral copper-containing catalysts, described below, are used.

An unexpected result of using an achiral or racemic catalyst to cyclize an optically inactive 4-trihalomethyl-substituted lactone is that, whereas there are two possible racemic pairs of the 4-trihalomethyl-substituted lactone (4 optical isomers), only one racemic pair is produced, the (1R,4R,5S) optical isomer and its mirror image.

It will be evident to those skilled in the art that other compounds capable of generating the carbenoid intermediates so derived from the aforesaid diazo derivatives of formula II can also be employed to produce bicyclic lactones of formula III.

Optically inactive copper-containing catalysts which may be employed to produce racemic lactones from racemic diazo derivatives of formula II or optically active lactones from optically active diazo derivatives include achiral or racemic coordination complexes of copper, for example, cupric acetylacetonate, cupric salicylaldehydeiminate, cupric ethylacetoacetate, as well as cuprous oxide, cupric oxide, cuprous chloride, cupric acetate, and metallic copper. Of these, achiral or racemic coordination complexes are preferred, and cupric acetylacetonate is especially preferred.

Optically active copper-containing catalysts to effect cyclization to an optically active lactone comprise chiral binuclear copper-containing catalysts selected from [N - (2 - hydroxyethylate)salicylideneaminato] - copper(II) binuclear complexes. Examples of these catalysts include optically active bis[2 - butoxy - $\alpha$ - (2 - butoxy - 5 - methylphenyl) - $\alpha$ - [1 - [[(2 - hydroxyphenyl)methylene]amino]-ethyl] - 5 - methylbenzenemethanolato (2 - )] - copper and bis[2 - butoxy - $\alpha$ - [2 - butoxy - 5 - (1,1-dimethylethyl)phenyl] - 5 - (1,1 - dimethylethyl) - $\alpha$ - [1 - [[2 - hydroxyphenyl)methylene]amino]ethyl]-benzenemethanolato (2 - )] - copper. Catalysts of (S) stereochemistry, for example, bis[2 - butoxy - 5-methylphenyl) - $\alpha$ - [(S) - 1 - [[(2 - hydroxy phenyl)methylene]amino]ethyl] - 5 - methylbenzenemethanolato (2 - )] - copper and bis[2 - butoxy - $\alpha$- [2 - butoxy - 5 - (1,1 - dimethylethyl)phenyl] - 5- (1,1 - dimethylethyl) - $\alpha$ - [(S) - 1 - [[2 - hydroxyphenyl)methylene]amino]ethyl]benzenemethanolato (2 - )] - copper, are preferred because they afford optically active lactones which can be carried, by processes described below, to *cis* - 3 - (2,2 - dihalovinyl) - 2,2 - dimethylcyclopropanecarboxylic acids

or esters, which are optically active due to enrichment with the (1R,3R) isomers. Among these catalysts, bis[2 - butoxy - α - [2 - butoxy - 5 - (1,1 - dimethylethyl)phenyl] - 5 - (1,1 - dimethylethyl)- α - [(S) - 1 - [[2 - hydroxyphenyl)methylene]amino]ethyl]benzenemethanolato (2 - )] - copper is especially preferred. Catalysts of (R) stereochemistry give rise ultimately to acids or esters which are rich in the (1S,3S) enantiomers. The catalysts may be prepared from optically active ethyl alanine by coupling with a Grignard reagent, producing a salicylaldimine from the resultant amino alcohol, and treating the imine with cupric acetate.

Aprotic solvents may be employed in the cyclization, for example, aromatic hydrocarbons and ethers, preferably toluene, benzene, n-octane, cyclohexane, or dioxane. In general, dioxane is preferred, but toluene is preferred for the tribromomethyl compounds. Improved yields are obtained if the reaction is conducted at high dilution.

In a fourth process embodiment, a bicyclic lactone of formula III, where $R^3$ is preferably methoxycarbonyl, is subjected to dealkoxycarbonylation, e.g., by treatment with lithium chloride in a polar solvent selected from hexamethylphosphoric triamide, dimethylformamide and dimethylsulfoxide, or mixtures thereof with water, cleaving the lower alkoxycarbonyl group and producing the corresponding lactone of formula

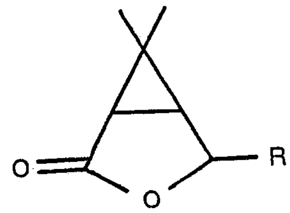

IV

The stereochemistry of the lactone is unaffected by dealkoxycarbonylation, and if the starting lactone is the (1R,4R,5S) isomer, the product is the (1R,4R,5S) isomer. A starting lactone produced as described above using a chiral catalyst of (S) stereochemistry affords a cleaved optically active lactone which can be carried, as described below, to *cis*-3-(2,2-dihalovinyl)-2,2-dimethylcyclopropanecarboxylic acids or esters, which are optically active due to enrichment with the (1R,3R) isomers.

In a fifth process embodiment, a bicyclic lactone of formula

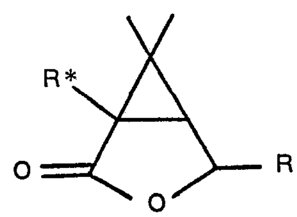

V

where R* is hydrogen or $R^3$ and R is hydrogen, is converted into a lower alkyl *cis*-3-(2,2-dihalovinyl)-2,2-dimethylcyclopropanecarboxylate. For example, 6,6-dimethyl-2-oxo-3-oxabicyclo[3.1.0]hexane is treated with base to open the lactone ring, followed by acidification and methylation with diazomethane, producing methyl *cis*-3-hydroxymethyl-2,2-dimethylcyclopropanecarboxylate. The alcohol is then oxidized to methyl cis-3-formyl-2,2-dimethylcyclopropanecarboxylate. A tri- or tetrahalomethane is then added to the methyl 3-formyl-2,2-dimethylcyclopropanecarboxylate, followed by acylation of the resulting 3-(1-hydroxy-1-trihalomethyl) derivative and reduction of the acylated compound with metallic zinc to afford a methyl 3-(2,2-dihalovinyl)-2,2-dimethylcyclopropanecarboxylate. It is preferred to employ the lactone having a hydrogen atom instead of the alkoxycarbonyl group $R^3$ since 6,6-dimethyl-2-oxo-3-oxabicyclo[3.1.0]hexane yields directly a lower alkyl *cis* ester which may be converted into a pyrethroid insecticide by methods well known in the art and referred to above. When a compound of Formula III is used the 1-alkoxycarbonylcyclopropanecarboxylate may be dealkoxycarbonylated and converted into a pyrethroid insecticide. Further, if an optically active lactone is employed, it is not only preferred that it be 6,6-dimethyl-2-oxo-3-oxabicyclo[3.1.0]hexane, but that the optical activity be due to enrichment with the (1R,5S) isomer, since an optically active cyclopropanecarboxylate enriched with the (1R,3R) isomer is then obtained. Optically active 6,6-dimethyl-2-oxo-3-oxabicyclo[3.1.0]hexane which is rich in the (1R,5S) isomer is obtained, for example, by the process embodiments described above, provided that the diazo ester is cyclized with a chiral binuclear copper-containing catalyst having (S) stereochemistry.

In a sixth process embodiment, a bicyclic lactone of the formula V where R is $CX_3$, is subjected to an elimination reaction, by treatment with a metal selected from zinc, magnesium, sodium, or coordination complexes of Cr(II), preferably in a solvent selected from acetic acid, alcohols such as ethanol, and ethers, such as tetrahydrofuran and diethyl ether, and mixtures thereof, thereby simultaneously opening the lactone ring, eliminating one halogen atom, and producing essentially free

of the *trans* isomer, a cis-3-(2,2-dihalovinyl)-2,2-dimethylcyclopropanecarboxylic acid, i.e., wherein the carboxy and dihalovinyl groups are *cis* with respect to each other. Preferably, the metal is zinc and the solvent is a mixture of an ether and acetic acid. It is preferred that R* be hydrogen, since the cyclopropanecarboxylic acid may then be converted directly into a pyrethroid insecticide as described above. In the case that R* is lower alkoxycarbonyl, the resultant 1-alkoxycarbonylcyclopropanecarboxylic acid may be dealkoxycarbonylated, for example, via the esters, and converted into a pyrethroid insecticide. Further, if an optically active lactone is employed, it is not only preferred that it be a 4-trihalomethyl-6,6-dimethyl-2-oxo-3-oxabicyclo[3.1.0]hexane, but that the optical activity be due to the (1R,4R,5S) isomer, since an optically active (1R,3R) cyclopropanecarboxylic acid is then obtained. An optically active (1R,4R,5S)-4-trihalomethyl-6,6-dimethyl-2-oxo-3-oxabicyclo[3.1.0]hexane is obtained, for example, by the process embodiments described above, provided that either the optically active (R) isomer of the diazo derivative of formula II is cyclized, or the diazo ester is cyclized with a chiral binuclear copper-containing catalyst having (S) stereochemistry.

In a preferred multistep process embodiment, a *cis*-3-(2,2-dihalovinyl)-2,2-dimethylcyclopropanecarboxylic acid, for example a (1R,3R) acid, is produced by treating a 3-methyl-1-trihalomethyl-2-butenyl acetoacetate, for example an (R) acetoacetate, of formula I with tosyl azide to effect diazo transfer, followed by sodium hydroxide to cleave the acetyl group and produce a 3-methyl-1-trihalomethyl-2-butenyl diazoacetate, for example an (R) diazoacetate, of formula II, which in turn is subjected to intramolecular carbenoid cyclization with cupric acetylacetonate to produce a 4-trihalomethyl-6,6-dimethyl-2-oxo-3-oxabicyclo[2.1.0]hexane, for example, a (1R,4R,5S)-bicyclohexane, of formula III, which is then treated with zinc and acetic acid to produce a *cis*-3-(2,2-dihalovinyl)-2,2-dimethylcyclopropanecarboxylic acid.

Although any compound of formulae I, II, and III may contain both chlorine and bromine and may be employed in the aforesaid processes, the trichloro and tribromo compounds are preferred, and the yields are generally higher with the chlorine-containing compounds. Thus, the processes disclosed above are especially well suited to preparing a *cis*-3-(2,2-dichlorovinyl)-2,2-dimethylcyclopropanecarboxylic acid or lower alkyl ester. The dibromovinyl acids or esters may be prepared in excellent yield by exchanging the halogen in the dichlorovinyl acid or a lower alkyl ester thereof.

The invention will be understood more completely by reference to the following preparative examples.

In the Examples which follow, temperatures are in degrees Celsius and pressures are in mm Hg. Tetramethylsilane was employed as an internal standard for the nmr spectra. In reporting the nmr data the abbreviations have the following significance: s, singlet; d, doublet; t, triplet; q, quartet; m, multiplet. Any of the abbreviations may be preceded by b for broad or d for double, for example, d.d., double doublet; b.t., broad triplet.

## Example I

### Synthesis of 4-Trihalomethyl-6,6-dimethyl-2-oxo-3-oxabicyclo[3.1.0]hexanes

A. From 3-methyl-1-trihalomethyl-2-butenyl diazoacetates

1. Preparation of 1,1,1-trichloro-4-methyl-3-penten-2-ol

To a solution of isobutene (89 g, 1.6 moles) and chloral (80 g, 0.52 mole) in about 100 ml of petroleum ether (bp, 30—70°), maintained at −5° to −10°, was added portionwise over a period of 30—60 minutes 6.08 g of anhydrous aluminum trichloride. The solution was stirred vigorously during the addition and thereafter for five hours at −5° to −10°, then overnight at room temperature.

After diluting the solution with ether, it was washed with 50—100 ml of water, dried over magnesium sulfate and distilled. After distillation of the petroleum ether, 1,1,1-trichloro-4-methyl-4-penten-2-ol (74.9 g, 71% yield) was obtained; bp, 106—118°/25 mm.

1,1,1-Trichloro-4-methyl-4-penten-2-ol (74 g, 0.36 mole) was heated at 140°—150° for 18 hours. After cooling, the alcohol was diluted with ether, and the ethereal solution was treated with activated charcoal. The ether was removed by evaporation to afford crystalline 1,1,1-trichloro-4-methyl-3-penten-2-ol (43.4 g, 59% yield) after recrystallization from petroleum ether.

1,1,1-Tribromo-4-methyl-3-penten-2-ol is similarly prepared by substituting bromal for chloral in the reaction.

1. (a) Preparation of (2R)-1,1,1-Trichloro-4-methyl-3-penten-2-ol

1,1,1-Trichloro-4-methyl-3-penten-2-yl (S)-N-(1-(1-naphthyl)ethyl)carbamate was prepared from racemic 1,1,1-trichloro-4-methyl-3-penten-2-ol. The diastereomer containing the (2R)-alcohol moiety was precipitated from the crude product by adding hexane, yielding the carbamate as a white solid, m.p. 120—123°.

The carbamate was cleaved with trichlorosilane to give (2R)-1,1,1-trichloro-4-methyl-3-penten-2-ol; $[\alpha]_D^{31} = -3.2°$ (hexane).

The absolute stereoconfigurations of the penten-2-ol and the compounds produced in Examples IA.2.(a), IA.4.(a), and IA.8.(a) below were established reasoning by analogy back through the sequence of reactions used to prepare the (1R,3R)-3-(2,2-dichlorovinyl)-2,2-dimethylcyclopropanecarboxylic acid in Example III C., the absolute stereoconfiguration of the acid being established independently.

2. Preparation of 3-methyl-1-trichloromethyl-2-butenyl acetoacetate

A mixture of 1,1,1-trichloro-4-methyl-3-penten-2-ol (20.4 g, 0.1 mole) and anhydrous sodium acetate (0.04 g) were heated together at 90—95° until the trichloro alcohol melted. Diketene (13.2 ml, 0.11 mole) was added dropwise in one hour to the mixture maintained at 80—83°. The mixture was then maintained at 80—83° for an additional two hours.

The reaction mixture was diluted with ether, and the ethereal solution was washed successively with aqueous 1N hydrochloric acid, saturated aqueous sodium bicarbonate, and saturated aqueous sodium chloride. The solution was then dried over magnesium sulfate, and the ether was evaporated, leaving a residue which was distilled under vacuum, affording 3-methyl-1-trichloromethyl-2-butenyl acetoacetate (25.3 g, 88% yield); bp, 95—96°/0.23 mm.

2. (a) Preparation of (1R)-3-Methyl-1-trichloromethyl-2-butenyl Acetoacetate

A mixture of (2R)- 1,1,1-trichloro-4-methyl-3-penten-2-ol (1.50 g, 7.35 mmole) from Example IA.1.(a) and a trace of sodium acetate was treated under nitrogen at 100° with freshly distilled diketene (0.70 g, 8.36 mmole) added dropwise over 1hr. After stirring and heating an additional hour at 80—83°, the cooled reaction mixture was diluted with 3 ml of $CH_2Cl_2$ and chromatographed on 8 (20 x 20 cm) preparative tlc plates, developed in $CH_2Cl_2$. Isolation with extraction afforded (1R)-3-methyl-1-trichloromethyl-2-butenyl acetoacetate (1.80 g, 85% isolated yield) as a pale yellow oil; $[\alpha]_D^{31} = +21.1°$. (hexane).

Analysis:

nmr $\delta$ ppm ($CHCl_3$): 6.08 (d, 1H), 5.34 (m, 1H), 3.53 (s, 2H), 2.30 (s, 3H), 1.87 (dd, 6H).

The nmr spectrum with chiral $Eu(hfc)_3$ showed only one half of the peak multiplicities that were found in nmr analysis of the racemate using the same reagent.

3. Preparation of 3-methyl-1-tribromomethyl-2-butenyl acetoacetate

1,1,1-Tribromo-4-methyl-3-penten-2-ol (6.72 g, 0.02 mole) and anhydrous sodium acetate (0.008 g) were melted together at 90°. The temperature of the mixture was then reduced to 80°, and diketene (about 2.6 ml, 0.022 mole) was added dropwise over a 10 minute period. The resulting mixture was heated at 80° for three additional hours. After cooling, the mixture was diluted with ether, and the ethereal solution was washed with 1N aqueous hydrochloric acid and then ten times with saturated aqueous sodium bicarbonate. The washed mixture was treated with activated charcoal and dried over magnesium sulfate. Evaporation of the ether afforded 3-methyl-1-tribromomethyl-2-butenyl acetoacetate (8.34 g, 99% yield) as an oily residue.

Analysis:

nmr $\delta$ ppm: 6.02 (d, 1H); 5.25 (m, 1H); 3.53 (s, 2H); 2.32 (s, 3H); 1.85 (m, 6H).

4. Preparation of 3-methyl-1-trichloromethyl-2-butenyl diazoacetate from 3-methyl-1-trichloromethyl-2-butenyl acetoacetate

To a solution of 3-methyl-1-trichloromethyl-2-butenyl acetoacetate (5.75 g, 0.02 mole) and triethylamine (2.02 g, 0.02 mole) in 50 ml of acetonitrile was added a solution of tosyl azide (3.94 g, 0.02 mole), in acetonitrile dropwise over a period of 10 minutes. The resultant mixture, containing the thus produced 3-methyl-1-trichloromethyl-2-butenyl-2-diazoacetoacetate, was stirred at room temperature for two hours and then poured into 60 ml (0.06 mole) of 1N aqueous sodium hydroxide. The resulting mixture was stirred at room temperature for 30 minutes and then extracted with about 100 ml of ether. After phase separation, the aqueous layer was extracted with an additional 150 ml portion of ether, and the ether extracts were combined. The ethereal solution was then washed ten times with 50 ml portions of 3% aqueous potassium hydroxide containing sodium chloride and then three times with 50 ml portions of saturated aqueous sodium chloride. The washed ethereal solution was dried over magnesium sulfate, and the ether was removed by evaporation, affording a brown oily residue. The residue was purified by column chromatography on silica gel using benzene as the eluent, to give 3-methyl-1-trichloromethyl-2-butenyl diazoacetate (4.63 g, 85% yield).

4. (a) Preparation of (1R)-3-Methyl-1-trichloromethyl-2-butenyl Diazoacetate

To a solution of (1R)-3-methyl-1-trichloromethyl-2-butenyl acetoacetate (1.63 g, 5.68 mmole) from Example IA.2.(a) and triethylamine (0.57 g, 5.68 mmole) in 10 ml of acetonitrile was added dropwise in small portions at 7—10 minute intervals over a total period of 30 minutes a solution of tosyl azide (1.12 g, 5.68 mmole) in 2 ml of acetonitrile. After stirring at room temperature for 2 1/4 hours, the mixture was poured into 17 ml of aqueous 1N NaOH. After stirring an additional 45 minutes, the mixture was extracted three times with ether, and the combined ethereal extracts were dried over $MgSO_4$. The dried solution was concentrated to give an orange oil. The oil was chromatographed on 8 (20 x 20 cm) preparative tlc plates (silica gel) with $CH_2Cl_2$ as eluent to afford (1R)-3-methyl-1-trichloromethyl-2-butenyl diazoacetate (0.96 g, 62% isolated yield) as a yellow oil; $[\alpha]_D^{31} = +59°$ (hexane).

5. Preparation of 3-methyl-1-tribromomethyl-2-butenyl diazoacetate

To a solution of 3-methyl-1-tribromomethyl-2-butenyl acetoacetate (8.32 g, 0.0198 mole) and tosyl azide (3.9 g, 0.0198 mole) in 40 ml of acetonitrile was added dropwise over a period of 35 minutes a solution of triethylamine (2.0 g, 0.020 mole) in 10 ml of acetonitrile. The resulting mixture was stirred at room temperature for 2 1/2 hours to give 3-methyl-1-tribromomethyl-2-butenyl acetodiazoacetate. Then 60 ml of 1N aqueous sodium hydroxide was added thereto, and the mixture was stirred at room temperature for 45 minutes. The reaction mixture was then diluted with ether, the organic phase was separated, the aqueous phase was extracted with ether, and the organic phases were combined. The ethereal solution was washed with three successive portions of 3% aqueous potassium hydroxide containing sodium chloride, then three times with saturated aqueous sodium chloride. The washed ethereal solution was dried over magnesium sulfate and treated with active charcoal. Evaporation of the ether afforded an oily residue. 3-Methyl-1-tribromomethyl-2-butenyl diazoacetate (5.78 g, 71% yield) was isolated therefrom by column chromatography.

Analysis:

nmr $\delta$ ppm ($CDCl_3$): 1.85 (dd, 6H); 4.82 (s, 1H); 5.33 (dq, 1H); 6.03 (d, 1H).

6. Preparation of 3-methyl-1-trichloromethyl-2-butenyl diazoacetate from the tosyl hydrazone of glyoxyoyl chloride

To a cold (0°) solution of the tosyl hydrazone of glyoxyoyl chloride (2.61 g, 0.01 mole) and 1,1,1-trichloro-4-methyl-3-penten-2-ol (2.035 g, 0.01 mole) in 25 ml of methylene chloride was added dropwise over a period of 20 minutes a solution of triethylamine (2.02 g, 0.02 mole) in 8 ml of methylene chloride. After stirring for one hour at 0°, the methylene chloride was evaporated at 25° under vacuum, leaving a residue. The residue was dissolved in 50 ml of benzene, and the dark brown solution was treated with activated charcoal and evaporated to give a yellow-brown oil. The oil was dissolved in methylene chloride, and the solution was washed successively twice with cold water, twice with cold aqueous sodium bicarbonate, and twice with cold water. The solution in methylene chloride was dried over sodium sulfate and evaporated at 25° under vacuum to give methyl-1-trichloromethyl-2-butenyl diazoacetate (2.67 g, 99% yield of crude product) as a viscous oil.

Analysis:

ir ($cm^{-1}$): 2970, 2950, 2900, 2130, 1770, 1740, 1700, 1440, 1340, 1310, 1270, 1200, 1170, 1095, 1070, 970, 840, 830, 800, 770, 750, 630, 560, 540, 450.

3-Methyl-1-trichloromethyl-2-butenyl diazoacetate, prepared similarly, except that the triethylamine was added in two portion an hour apart, the resulting mixture was then stirred for 2.5 hours at 0°, and the product was purified by column chromatography, displayed the following nmr spectrum.

nmr $\delta$ ppm ($CCl_4$): 5.93 (d, 1H); 5.23 (dq, 1H); 4.72 (s, 1H); 1.87 (t, 6H).

7. Preparation of 4-trichloromethyl-6,6-dimethyl-2-oxo-3-oxabicyclo[3.1.0]hexane

To a refluxing solution of cupric acetylacetonate (0.07 g, 0.27 mole) in 70 ml of dioxane was added dropwise over a 3 1/2 hour period, under an argon atmosphere, a solution of 3-methyl-1-trichloromethyl-2-butenyl diazoacetate (1.09 g, 0.004 moles) and cupric acetylacetonate (0.007 g, 0.027 mmole) in 10 ml of dioxane. The reaction mixture was then heated under reflux for one hour and evaporated to dryness, yielding a residue which was dissolved in ether. The ethereal solution was washed successively with two portions of aqueous 1N hydrochloric acid, two portions of aqueous sodium bicarbonate, and twice with saturated aqueous sodium chloride. After drying the solution over magnesium sulfate, the ether was evaporated to afford a pale yellow oil, which was purified by column chromatography on a silica gel column using a 9/1 mixture of n-hexane/ethyl acetate as the eluent, to give 4-trichloromethyl-6,6-dimethyl-2-oxo-3-oxabicyclo[3.1.0]hexane (0.55 g, 53% yield).

8. Preparation of optically active 4-trichloromethyl-6,6-dimethyl-2-oxo-3-oxabicyclo[3.1.0]hexane

A solution of 3-methy-1-trichloromethyl-2-butenyl-diazoacetate (0.544 g, 2 mmole) in dioxane (5 ml) was added dropwise over a period of about 4.5 hours to a suspension of bis - [2 - butoxy - $\alpha$-[2 - butoxy - 5 - (1,1 - dimethylethyl)phenyl] - 5 - (1,1 - dimethylethyl) - $\alpha$ - [(S) - 1 - [[(2 - hydroxyphenyl)methylene]amino]ethyl]benzenemethanolato - (2 - )] copper (0.762 g, 0.05 mmole) in dioxane (35 ml) at 100—105°. When the evolution of nitrogen began, the temperature was slowly lowered to 50° and maintained at 50° throughout the addition. After the addition of the 3-methyl-1-trichloromethyl-2-butenyl diazoacetate was completed, the reaction mixture was stirred at 50° for 2 hours. The reaction mixture was then evaporated to dryness under vacuum and the residue dissolved in diethyl ether. The ethereal solution was washed with a 1N aqueous solution of hydrochloric acid until the washings did not turn blue when tested with aqueous ammonia, then washed once with a saturated aqueous solution of sodium chloride and dried over magnesium sulfate. The ether was evaporated under vacuum, and the crude 4-trichloromethyl-6,6-dimethyl-2-oxo-3-oxabicyclo[3.1.0]hexane enriched with the (1R,4R,5S) isomer (0.47 g) was converted by zinc reduction to 3-(2,2-dichlorovinyl)-2,2-dimethylcyclopropanecarboxylic acid. The acid, after purification by column chromatography, had an optical rotation of $[\alpha]_D^{18.5} = +4.7°$ ($CHCl_3$). The optical yield of (1R,3R)-3-(2,2-dichlorovinyl)-2,2-dimethylcyclopropanecarboxylic acid from the 3-methyl-1-trichloromethyl-2-butenyl diazoacetate was calculated to be about 14%.

Optically active 4-tribromomethyl-6,6-dimethyl-2-oxo-3-oxabicyclo[3.1.0]hexane enriched with the (1R,4R,5S) isomer is similarly prepared from 3-methyl-1-tribromomethyl-2-butenyl diazoacetate.

Optically active lactones enriched with the (1S,4S,5R) isomers are prepared by substituting the (R) catalyst for the (S) catalyst specified above.

8. (a) Preparation of (1R,4R,5S)-6,6-Dimethyl-2-oxo-4-trichloromethyl-3-oxabicyclo[3.1.0]hexane

A solution of (1R)-3-methyl-1-trichloromethyl-2-butenyl diazoacetate (0.90 g, 3.33 mmole) from Example IA.4.(a) in 15 ml of dry dioxane was added dropwise over a period of 2.6 hours to a catalytic amount (approximately 2 mole %) of cupric acetylacetonate in 65 ml of refluxing dry dioxane under a nitrogen atmosphere. After refluxing for an additional hour, the mixture was evaporated, and an etheral solution of the residue was washed with 1N HCl, saturated $NaHCO_3$, and saturated NaCl, then dried over $MgSO_4$ and evaporated to give (1R,4R,5S)-6,6-dimethyl-2-oxo-4-trichloromethyl-3-oxabicyclo-[3.1.0]hexane (0.73 g, 90% crude yield) as a green-yellow oil.

Analysis of the nmr spectrum of the product with $Eu(hfc)_3$ gave two singlets for the methyl region, Analysis of the racemate by nmr with $Eu(hfc)_3$ showed two well-defined doublets in the methyl region, with other multiplicities also visible.

9. Preparation of 4-tribromomethyl-6,6-dimethyl-2-oxo-3-oxabicyclo[3.1.0]hexane

To a refluxing solution of cupric acetylacetonate (0.005 g, 0.019 mmole) in 40 ml of toluene was added dropwise over a period of 2.5 hours under an atmosphere of argon gas a solution of 3-methyl-1-tribromomethyl-2-butenyl diazoacetate (0.203 g, 0.005 ml) in 10 ml of toluene. The mixture was then heated under reflux for one hour. After cooling, the reaction mixture was washed successively with two portions of 1N aqueous hydrochloric acid, once with saturated aqueous sodium bicarbonate, and twice with saturated aqueous sodium chloride. After drying over magnesium sulfate, the solution was evaporated to give a yellow oil as the residue. The residue was purified by column chromatography on a silica gel column using 9/1 n-hexane/ethyl acetate as the eluent, yielding 4-tribromomethyl-6,6-dimethyl-2-oxo-3-oxabicyclo[3.1.0]hexane (0.053 g, yield 28%); mp 101—103° after recrystallization from n-hexane.

Analysis:

Calculated for $C_8H_9Br_3O_2$: C, 25.50; H, 2.41;

Found: C, 25.52; H, 2.36.

nmr $\delta$ ppm ($CDCl_3$): 1.28 (2s, 6H); 2.18 (d, 1H) 2.37 (d, 1H); 4.58 (s, 1H)

The nmr spectrum indicates that the lactone is the (1S,4S,5R)/(1R,4R,5S) racemic mixture.

10. Preparation of 1-trichloromethyl-3-methyl-2-butenyl-2-acetodiazoacetate

To a solution of 1-trichloromethyl-3-methyl-2-butenyl acetoacetate (0.575 g, 2 mmole) and triethylamine (0.404 g, 4 mmole) in 10 ml of acetonitrile was added 1 g of polymer-bound sulfonyl azide. The mixture was stirred overnight at room temperature and filtered. The filter cake was washed with ether, and the slurry was filtered. Solvent was removed by evaporation from the combined filtrates to give 1-trichloromethyl-3-methyl-2-butenyl 2-acetodiazoacetate (0.60 g, 96% yield).

Analysis:

nmr $\delta$ ppm: 6.03 (d, 1H); 5.28 (bd, 1H); 2.45 (s, 3H); 1.90 (d, 6H).

B. From lower alkyl 3-methyl-1-trichloromethyl-2-butenyl diazomalonates

1. Preparation of ethyl 3-methyl-1-trichloromethyl-2-butenyl malonate

To a solution of 1,1,1-trichloro-4-methyl-3-penten-2-ol (7.98 g, 0.0392 mole) and pyridine (3.10 g, 0.0392 mole) in 8 ml of ether was added dropwise a solution of ethyl malonyl chloride (5.87 g, 0.039 mole) in 4 ml of ether. The reaction mixture was then stirred overnight at room temperature.

The reaction mixture was diluted with ether and then washed with aqueous 1N hydrochloric acid, then aqueous sodium bicarbonate, followed by aqueous sodium chloride. The washed mixture was dried over magnesium sulfate, the ether was removed by evaporation, and the residue was distilled to afford ethyl 3-methyl-1-trichloromethyl-2-butenyl malonate (5.68 g, 46% yield); bp, 104—105°/0.3 mm.

Analysis:

nmr $\delta$ ppm: 6.00 (d, 1H); 5.32 (dq, 1H); 4.18 (dd, 2H); 3.35 (s, 2H); 1.88 (m, 6H); 1.30 (t, 3H).

2. Preparation of ethyl 3-methyl-1-trichloromethyl-2-butenyl diazomalonate

To a solution of ethyl 3-methyl-1-trichloromethyl-2-butenyl malonate (5.63 g, 0.0177 mole) and triethylamine (1.79 g, 0.0177 mole) in 40 ml of acetonitrile was added dropwise a solution of tosyl azide (3.487 g, 0.0177 mole) in 10 ml of acetonitrile. After the addition, the mixture was stirred overnight at room temperature.

The acetonitrile was then evaporated from the solution at room temperature under vacuum, yielding a residue, which was dissolved in ether. The ethereal solution was washed successively with

three portions of 3% aqueous potassium hydroxide and once with aqueous sodium chloride. After drying the solution over magnesium sulfate, the ether was evaporated to yield ethyl 3-methyl-1-trichloromethyl-2-butenyl diazomalonate (5.91 g, 85% yield) as the residue.

Analysis:

nmr δ ppm ($CCl_4$): 6.03 (d, 1H); 5.30 (dq, 1H); 4.27 (dd, 2H); 1.88 (m, 6H); 1.33 (t, 3H).

3. Preparation of ethyl 4-trichloromethyl-6,6-dimethyl-2-oxo-3-oxabicyclo[3.1.0]hexane-1-carboxylate

To a refluxing suspension of copper powder (5.0 g, 0.079 mole) in 240 ml of n-octane was added dropwise in one hour a solution of ethyl 3-methyl-1-trichloromethyl-2-butenyl diazomalonate (5.0 g, 0.0146 mole) in 60 ml of n-octane. The reaction mixture was then heated under reflux for 2 1/2 hours and filtered. The n-octane was removed from the filtrate by evaporation under vacuum, yielding a residue which was distilled under vacuum to give ethyl 4-trichloromethyl-6,6-dimethyl-2-oxo-3-oxabicyclo[3.1.0]hexane-1-carboxylate as a highly viscous oil (2.47 g, 54% yield); bp, 123—125°/0.3 mm.

Analysis:

nmr δ ppm ($CCl_4$): 4.47 (s, 1H); 4.23 (dd, 2H); 2.67 (s, 1H); 1.32 (m, 9H).

4. Preparation of methyl 4-trichloromethyl-6,6-dimethyl-2-oxo-3-oxabicyclo[3.1.0]hexane-1-carboxylate

To a refluxing suspension of copper powder (6.6 g, 0.104 mole) in 320 ml of n-octane was added dropwise in one hour a solution of methyl 3-methyl-1-trichloromethyl-2-butenyl diazomalonate (6.6 g, 0.02 mole, prepared as described above for the ethyl ester) in 80 ml of n-octane. The reaction mixture was heated for 2 1/2 hours under reflux and then filtered while hot. The n-octane was evaporated from the filtrate under vacuum to produce a residue which was dissolved in ethyl acetate. The solution was washed successively with aqueous 1N hydrochloric acid, aqueous sodium bicarbonate, and aqueous sodium chloride. After drying the solution over magnesium sulfate, the ethyl acetate was removed by evaporation, yielding methyl 4-trichloromethyl-6,6-dimethyl-2-oxo-3-oxabicyclo[3.1.0]hexane-1-carboxylate (5.77 g, 96% yield) as an oil. Trituration of the oil with ether afforded the crystalline lactone; mp, 118—120°.

Analysis:

| | | |
|---|---|---|
| Calculated for $C_{10}H_{11}O_4Cl_3$: | C, 39.83; | H, 3.68; |
| Found: | C, 39.57; | H, 3.56. |

nmr δ ppm ($CDCl_3$): 4.39 (s, 1H); 3.76 (s, 3H); 2.61 (s, 1H); 1.30 (s, 6H).

mass spec. m/e: 300, 109, 100, 33, 18.

molecular weight ($CHCl_3$): 295

The nmr spectrum indicates that the lactone is the (1S,4S,5R)/(1R,4R,5S) racemic mixture.

5. Preparation of 4-trichloromethyl-6,6-dimethyl-2-oxo-3-oxabicyclo[3.1.0]hexane

To a solution of methyl 4-trichloromethyl-6,6-dimethyl-2-oxo-3-oxabicyclo[3.1.0]hexane-1-carboxylate (1.52 g, 0.005 mole) in 10 ml of hexamethylphosphoric triamide was added lithium chloride (0.42 g, 0.010 mole). After heating at 75° for two hours, the cooled reaction mixture was diluted with ether. The ethereal solution was washed with water, dried over magnesium sulfate, and the ether was removed by evaporation, yielding a residue which was purified by column chromatography on silica gel using 9/1 n-hexane/ethylacetate as the eluent to give 4-trichloromethyl-6,6-dimethyl-2-oxo-3-oxabicyclo[3.1.0]hexane (0.34 g, 28% yield) as an oil.

Analysis:

nmr δ ppm ($CDCl_3$): 4.60 (s, 1H); 2.37 (d, 1H); 2.15 (d, 1H); 1.28 (s, 6H).

mass spec. m/e: 243, 125, 100, 97, 30.

Following the above procedure, the same compound is prepared from ethyl 4-trichloromethyl-6,6-dimethyl-2-oxo-3-oxabicyclo[3.1.0]hexane-1-carboxylate.

## Example II

Synthesis of 6,6-Dimethyl-2-oxo-3-oxabicyclo[3.1.0]hexane

1. Preparation of 3-methyl-2-butenyl acetoacetate

A mixture of 3-methyl-2-buten-1-ol (21.2 g, 0.25 mole) and anhydrous sodium acetate (0.1 g) was cooled to 0°. Diketene (36 ml, 0.3 mole) was added dropwise over a period of 1.5 to 2 hours. The reaction mixture was then heated to 75—80°, and this temperature was maintained for one hour. The mixture was diluted with ether. The ethereal solution was washed succesively with aqueous 1N hydrochloric acid, saturated aqueous sodium bicarbonate, saturated aqueous sodium chloride, and then dried over magnesium sulfate. The ether was evaporated, leaving a residue which was distilled under vacuum, affording 3-methyl-2-butenyl acetoacetate (32.1 g, 76% yield); bp 114—117°/18—19 mm.

2. Preparation of 3-methyl-2-butenyl diazoacetate

A solution of 3-methyl-2-butenyl acetoacetate (7.4 g, 0.0435 mole) and p-toluenesulfonyl azide

was prepared in acetonitrile. Triethylamine (4.40 g) in acetonitrile was added dropwise to this solution over a period of 10 minutes, and the stirred reaction mixture was maintained at room temperature for 2 hours. An aqueous 1N solution of sodium hydroxide (130 ml, 0.13 mole) was added and stirring continued for 45 minutes. Ether and aqueous sodium chloride were added to the mixture. After phase separation, the aqueous layer was washed consecutively with aqueous 1N sodium hydroxide (3 times) and a saturated aqueous solution of sodium chloride (twice). The washed ethereal solution was dried over magnesium sulfate and treated with active charcoal. The residue, after evaporation, was identified as 3-methyl-2-butenyl diazoacetate (5.72 g, 85% yield).

Analysis:

nmr $\delta$ ppm: 1.8 (m, 6H); 4.6 (s, 1H); 4.8 (m, 2H); 5.4 (m, 1H).

3. Preparation of 6,6-dimethyl-2-oxo-3-oxabicyclo[3.1.0]hexane

A solution of 3-methyl-2-butenyl diazoacetate (41.3 mmole, 6.36 g) in dioxane (20 ml) was added dropwise to a refluxing solution of cupric acetylacetonate (1.03 mmole, 0.27 g) in dioxane (400 ml) over a period of 7 hours under an argon atmosphere. After completion of the addition, the reaction mixture was heated under reflux for 1 hour. The crude reaction mixture was distilled to afford 6,6-dimethyl-2-oxo-3-oxabicyclo[3.1.0]hexane (3.85 g, 75% yield); bp 110°, 16 mm Hg.

4. Preparation of optically active 6,6-dimethyl-2-oxo-3-oxabicyclo[3.1.0]hexane

A solution of 3-methyl-2-butenyl diazoacetate (980 mg, 6.4 mmoles) in dioxane (15 ml) was added dropwise to a solution of bis[2 - butoxy - $\alpha$ - [2 - butoxy - 5 - (1,1 - dimethylethyl)phenyl] - 5-(1,1 - dimethylethyl) - $\alpha$ - [(S) - 1 - [[2 - hydroxyphenyl)methylene]amino]ethyl]benzenemethanolato (2 - )] - copper (83 mg, 0.064 mmole) in dioxane (55 ml) at 110°. When the evolution of nitrogen started, the temperature was slowly lowered to 52—3°. The addition took 5.5 hours, and the evolution of almost theoretical amounts of nitrogen was observed. After the addition was completed, the reaction mixture was stirred at 52—3° for 2 hours, evaporated to dryness, and distilled under vacuum to afford 6,6-dimethyl-2-oxo-3-oxabicyclo[3.1.0]hexane enriched with the (1R,5S) isomer (500 mg). The optical rotation of the product was $[\alpha]_D^{29} = -53.5°$ ($CHCl_3$).

The product was then treated with base, acidified, then methylated, before oxidation to methyl *cis*-3-formyl-2,2-dimethylcyclopropanecarboxylate. The latter was then converted to a lower alkoxy 3-(2,2-dichlorovinyl)-2,2-dimethylcyclopropanecarboxylate. The ester was hydrolyzed, and the optical activity of the resulting *cis*-3-(2,2-dichlorovinyl)-2,2-dimethylcyclopropanecarboxylic acid was $[\alpha]_D = +16.7$ ($CHCl_3$), corresponding to an optical yield of about 58% (1R,3R) isomer.

## Example III

### Synthesis of *cis*-3-(2,2-Dihalovinyl)-2,2-dimethylcyclopropanecarboxylic Acids

A. Preparation of *cis*-3-(2,2-dichlorovinyl)-2,2-dimethylcyclopropanecarboxylic acid

To a suspension of zinc powder (0.52 g, 0.008 mole) in 0.6 ml of acetic acid and 3 ml of ether was added dropwise over a period of 15 minutes a solution of 4-trichloromethyl-6,6-dimethyl-2-oxo-3-oxabicyclo[3.1.0]hexane (0.487 g, 0.002 mole) in 3 ml of ether. The mixture was stirred for two hours at room temperature, 30 ml of ether and 5 ml of water were added, and the mixture was then filtered through a filter aid. The organic layer was separated, washed twice with saturated aqueous sodium chloride, dried over magnesium sulfate, and the ether was evaporated to give crystalline *cis*-3-(2,2-dichlorovinyl)-2,2-dimethylcyclopropanecarboxylic acid (0.405 g, 97% yield).

Analysis:

nmr $\delta$ ppm ($CDCl_3$): 1.18 (s, 6H); 1.92 (m, 2H); 6.15 (d, 1H); 13.27 (bs, 1H).

The presence in the nmr spectrum of the single doublet at 6.15 ppm indicates that only the *cis* isomer is present.

B. Preparation of *cis*-3-(2,2-dibromovinyl)-2,2-dimethylcyclopropanecarboxylic acid

To a chilled suspension of zinc powder (0.494 g, 0.0076 mole) in 0.57 ml of acetic acid and 3 ml of ether was added dropwise over a period of 20 minutes a solution of 4-tribromomethyl-6,6-dimethyl-2-oxo-3-oxabicyclo[3.1.0]hexane (0.72 g, 0.0019 mole) in 5 ml of ether. The mixture was stirred at 0° for one hour. After adding 30 ml of ether and 5 ml of water, the mixture was filtered through a filter aid. The organic layer was separated, washed three times with saturated aqueous sodium chloride, dried over magnesium sulfate, and the ether was removed by evaporation to give crystalline *cis*-3-(2,2-dibromovinyl)-2,2-dimethylcyclopropanecarboxylic acid (0.489 g, 86% yield); mp, 112—114° after recrystallization from n-hexane.

Analysis:

nmr $\delta$ ppm ($CDCl_3$): 1.27 (s, 6H); 1.97 (m, 2H); 6.67 (d, 1H); 11.3 (bs, 1H).

The presence in the nmr spectrum of the single doublet at 6.67 ppm shows that only the *cis* isomer is present.

If an optically active 4-trihalomethyl-6,6-dimethyl-2-oxo-3-oxabicyclo[3.1.0]hexane is employed, for example, enriched with the (1R,4R,5S) optical isomer, an optically active *cis*-3-(2,2-

dihalovinyl)-2,2-dimethylcyclopropanecarboxylic acid, for example, enriched with the (1R,3R) isomer, is obtained.

C. Preparation of (1R,3R)-3-(2,2-Dichlorovinyl)-2,2-dimethylcyclopropanecarboxylic Acid

To a suspension of finely divided zinc dust (0.81 g, 1.23 mmole) in acetic acid (0.99 g, 1.64 mmole) and 4 ml of ether was added, under nitrogen, a solution of (1R,4R,5S)-6,6-dimethyl-2-oxo-4-trichloromethyl-3-oxabicyclo[3.1.0]hexane (0.50 g, 1.84 mmole) from Example IA.8.(a) in 4 ml of ether. The reaction was monitored by glpc. When the glpc results indicated almost complete conversion, the reaction mixture was diluted with additional ether and water, filtered through a filter aid, and the organic layer was separated, dried, and evaporated to a yellow oil which was chromatographed on two 20 x 20 cm preparative tlc plates with $CH_2Cl_2$ as eluent. The slow running acid band was removed, extracted with $CH_2Cl_2$, and the extract was filtered and evaporated to afford (1R,3R)-3-(2,2-dichlorovinyl)-2,2-dimethylcyclopropanecarboxylic acid. (68 mg, 18% isolated yield). The optical rotation of the acid prepared in a second identical run was measured; $[\alpha]_D^{31} = +6.2$ (hexane).

To the aforesaid cyclopropanecarboxylic acid (65 mg, 0.325 mmole) sealed in a glpc autosampler vial (capacity ~2 ml) was added by syringe all at once thionyl chloride (194 mg, 1.63 mmole). The vial was suspended in a 45° oil bath for 2 1/4 hr, after which 0.2 ml of $Et_2O$ was added, and the solvent and excess thionyl chloride were removed by syringe under high vacuum. To the residue was added 0.5 ml of toluene, after which several drops of the resultant solution was added to a second sealed glpc autosample vial containing 1 drop of (—)-2-octanol, 1 drop of pyridine, and 0.1 ml of toluene. The vial was suspended in a 45° oil bath for 1 hr, during which a white precipitate formed. After 1 hour, the supernatant liquid was analyzed by glpc on a 12′ x 1/8″ stainless steel column packed with 15% QF—1 on Chrom W. The chromatogram indicated that the ester produced in the reaction was at least 93% (—)-2-octanol ester of (1R,3R)-3-(2,2-dichlorovinyl)-2,2-dimethylcyclopropanecarboxylic acid, based on a comparison of the retention times of the product and an authentic sample of the (—)-2-octanol ester of (1R,3R)-3-(2,2-dichlorovinyl)-2,2-dimethylcyclopropanecarboxylic acid.

In the manner of Examples IA.1.(a), IA.2.(a), IA.4.(a), IA.8.(a), and III C., (1S,3S)-3-(2,2-dichlorovinyl)-2,2-dimethylcyclopropanecarboxylic acid is prepared from (2S)-1,1,1-trichloro-4-methyl-3-penten-2-ol via (1S,4S,5R)-6,6-dimethyl-2-oxo-4-trichloromethyl-3-oxabicyclo[3.1.0]hexane.

## Claims

1. A compound of the formula:

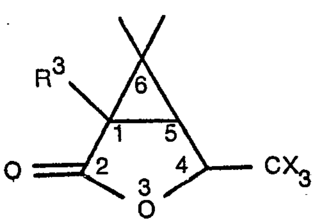

in which $R^3$ is ($C_{1-6}$ alkoxy)carbonyl and each X, which is the same as or different to the others, is a chlorine or bromine atom.

2. Ethyl 4-trichloromethyl-6,6-dimethyl-2-oxo-3-oxabicyclo[3.1.0]hexane-1-carboxylate.

3. Methyl 4-trichloromethyl-6,6-dimethyl-2-oxo-3-oxabicyclo[3.1.0]hexane-1-carboxylate.

4. A compound of the formula:

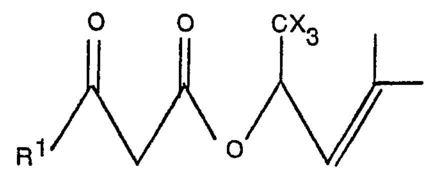

in which $R^1$ is $C_{1-6}$ alkyl or $C_{1-6}$ alkoxy and each X, which is the same as or different from the others, is a chlorine or bromine atom.

5. A compound as claimed in Claim 4 in which $R^1$ is $C_{1-6}$ alkyl.

6. 3-Methyl-1-trichloromethyl-2-butenyl acetoacetate.

7. (1R)-3-Methyl-1-trichloromethyl-2-butenyl acetoacetate.

8. 3-Methyl-1-tribromomethyl-2-butenyl acetoacetate.

9. A compound as claimed in Claim 4 in which $R^1$ is $C_{1-6}$ alkoxy.

10. Ethyl 3-methyl-1-trichloromethyl-2-butenyl malonate.

11. A compound of the formula:

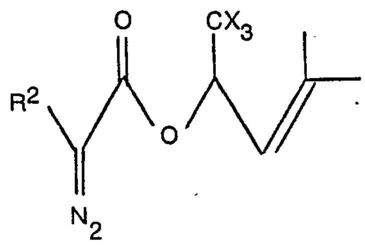

in which $R^2$ is hydrogen, ($C_{1-6}$ alkoxy)carbonyl, of $C_{1-6}$ alkanoyl and each X, which is the same as or different from the others, is a chlorine or bromine atom.

12. A compound as claimed in Claim 11 in which $R^2$ is hydrogen.

13. 3-Methyl-1-trichloromethyl-2-butenyl diazoacetate.

14. (1R)-3-Methyl-1-trichloromethyl-2-butenyl diazoacetate.

15. 3-Methyl-1-tribromomethyl-2-butenyl diazoacetate.

16. A compound as claimed in Claim 11 in which $R^2$ is ($C_{1-6}$ alkoxy)carbonyl.

17. Ethyl 3-methyl-1-trichloromethyl-2-butenyl diazomalonate.

18. Methyl 3-methyl-1-trichloromethyl-2-butenyl diazomalonate.

19. A compound as claimed in Claim 11 in which $R^2$ is $C_{1-6}$ alkanoyl.

20. 1-Trichloromethyl-3-methyl-2-butenyl acetodiazoacetate.

21. A process for preparing a lactone of the formula:

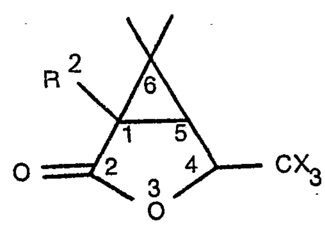

characterized by cyclizing a diazo derivative of the formula:

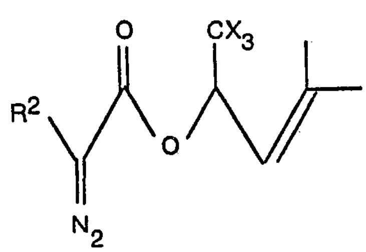

by intramolecular carbenoid cyclization, where each X is a chlorine or bromine atom and $R^2$ is hydrogen or ($C_{1-6}$ alkoxy)carbonyl.

22. A process as claimed in Claim 21 characterized by cyclizing the diazo derivative with a copper-containing catalyst.

23. A process as claimed in Claim 22 characterized in that the catalyst is a chiral binuclear copper-containing catalyst.

24. A process as claimed in Claim 23 characterized in that the catalyst is a [N-(2-hydroxyethylate)salicylideneaminato]copper (II) binuclear complex.

25. A process as claimed in Claim 24 characterized in that the catalyst is bis{2 - butoxy - $\alpha$ - [2 - butoxy - 5 - (1,1 - dimethylethyl) - phenyl] - 5 - (1,1 - dimethylethyl) - $\alpha$ - [(S) - 1 - ([(2 - hydroxyphenyl)methylene] - amino)ethyl]benzene methanolato(2 - )} - copper.

26. A process as claimed in Claim 25 characterized in that 4-trichloromethyl-6,6-dimethyl-2-oxo-3-oxabicyclo[3.1.0]hexane enriched with the (1R,4R,5S) isomer is prepared from 3-methyl-1-trichloromethyl-2-butenyl diazoacetate.

27. A process as claimed in Claim 22 characterized in that the diazo derivative and lactone are optically active.

28. A process as claimed in Claim 27 characterized in that (1R,4R,5S)-4-trichloromethyl-6,6-dimethyl-2-oxo-3-oxabicyclo[3.1.0]hexane substantially free from other isomers thereof is prepared from (R)-3-methyl-1-trichloromethyl-2-butenyl diazoacetate.

29. A process as claimed in Claim 22 characterized in that 4-trichloromethyl-6,6-dimethyl-2-oxo-3-oxabicylo[3.1.0]hexane is prepared from 3-methyl-1-trichloromethyl-2-butenyl diazoacetate.

30. A process as claimed in Claim 22 characterized in that 4-tribromomethyl-6,6-dimethyl-2-oxo-3-oxabicyclo[3.1.0]hexane is prepared from 3-methyl-1-tribromomethyl-2-butenyl diazoacetate.

31. A process as claimed in Claim 22 characterized in that ethyl 4-trichloromethyl-6,6-dimethyl-2-oxo-3-oxabicyclo[3.1.0]hexane-1-carboxylate is prepared from ethyl 3-methyl-1-trichloromethyl-2-butenyl diazomalonate.

32. A process as claimed in Claim 22 characterized in that methyl 4-trichloromethyl-6,6-dimethyl-2-oxo-3-oxabicyclo[3.1.0]hexane-1-carboxylate is prepared from methyl 3-methyl-1-trichloromethyl-2-butenyl diazomalonate.

33. A process as claimed in any one of Claims 21 to 32 characterized in that the lactone so prepared is treated with a metal selected from zinc, magnesium, sodium, and coordination complexes of Cr(II) in solvent selected from acetic acid, alcohols, and ethers and mixtures thereof, producing a compound of the formula:

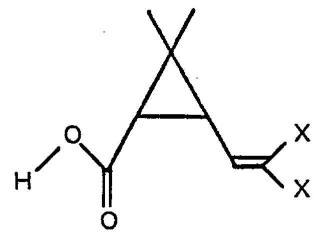

in which the carboxy and dihalovinyl groups are *cis* with respect to each other.

34. A process as claimed in Claim 33 characterized in that *cis*-3-(2,2-dichlorovinyl)-2,2-dimethylcyclopropanecarboxylic acid is prepared from 4-trichloromethyl-6,6-dimethyl-2-oxo-3-oxabicyclo[3.1.0]hexane.

35. A process as claimed in Claim 33 characterized in that (1R,3R)-3-(2,2-dichlorovinyl)-2,2-dimethylcyclopropanecarboxylic acid is prepared from (1R,4R,5S)-6,6-dimethyl-2-oxo-4-trichloromethyl-3-oxabicyclo[3.1.0]hexane.

36. A process as claimed in Claim 33 characterized in that *cis*-3-(2,2-dibromovinyl)-2,2-dimethylcyclopropanecarboxylic acid is prepared from 4-tribromomethyl-6,6-dimethyl-2-oxo-3-oxabicyclo[3.1.0]hexane.

37. A process as claimed in any one of Claims 21 to 25 and 27 in which $R^2$ is ($C_{1-6}$ alkoxy)carbonyl, characterized in that the resulting lactone is dealkoxycarbonylated to form the corresponding lactone in which $R^2$ is hydrogen.

38. A process as claimed in Claim 37 characterized in that the dealkoxycarbonylation is carried out with lithium chloride in a polar solvent.

39. A process as claimed in Claim 38 characterized in that, in the dealkoxycarbonylation step, 4-trichloromethyl-6,6-dimethyl-2-oxabicyclo[3.1.0]hexane is prepared from methyl or ethyl 4-trichloromethyl-6,6-dimethyl-2-oxo-3-oxabicyclo[3.1.0]hexane-1-carboxylate.

40. A process as claimed in Claim 21 characterized in that the diazo derivative of the formula:

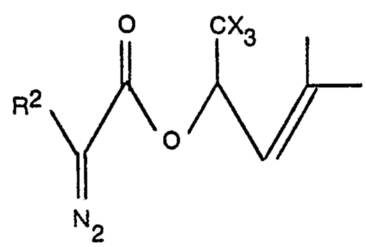

is prepared from an ester of the formula:

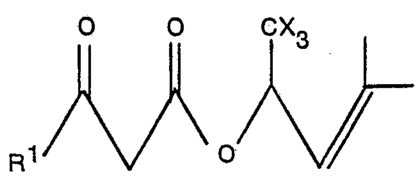

in which each X is a chlorine or bromine atom, $R^1$ is $C_{1-6}$ alkyl or $C_{1-6}$ alkoxy, and $R^2$ is hydrogen or ($C_{1-6}$ alkoxy)carbonyl, by reacting the ester with an azide.

41. A process as claimed in Claim 40 characterized in that a diazo derivative in which $R^2$ is hydrogen is prepared by treating an ester in which $R^1$ is $C_{1-6}$ alkyl with an azide and then with base.

42. A process as claimed in Claim 41 characterized in that 3-methyl-1-trichloromethyl-2-butenyl

diazoacetate is prepared from 3-methyl-1-trichloromethyl-2-butenyl acetoacetate.

43. A process as claimed in Claim 41 characterized in that 3-methyl-1-tribromomethyl-2-butenyl diazoacetate is prepared from 3-methyl-1-tribromomethyl-2-butenyl acetoacetate.

44. A process as claimed in Claim 41 characterized in that (1R)-3-methyl-1-trichloromethyl-2-butenyl diazoacetate is prepared from (1R)-3-methyl-1-trichloromethyl-2-butenyl acetoacetate.

45. A process as claimed in Claim 40 characterized in that a diazo derivative in which $R^2$ is ($C_{1-6}$ alkoxy)carbonyl is prepared by treating with an azide an ester in which $R^1$ is $C_{1-6}$ alkoxy.

46. A process as claimed in Claim 45 characterized in that ethyl 3-methyl-1-trichloromethyl-2-butenyl diazomalonate is prepared from ethyl 3-methyl-1-trichloromethyl-2-butenyl malonate.

47. A process as claimed in Claim 40 characterized in that a diazo ester of formula:

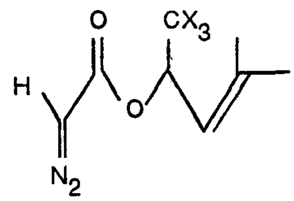

is prepared by reacting an alcohol of formula:

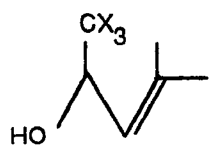

with a hydrazone of glyoxylyl chloride, X being as defined in Claim 1.

48. A process as claimed in Claim 47 characterized in that 3-methyl-1-trichloromethyl-2-butenyl diazoacetate is prepared by reacting 1,1,1-trichloro-4-methyl-3-penten-2-ol with the *p*-toluenesulfonyl hydrazone of glyoxyloyl chloride.

49. 3-Methyl-2-butenyl diazoacetate.

50. The process that comprises cyclizing 3-methyl-2-butenyl diazoacetate by intramolecular carbenoid cyclization to produce 6,6-dimethyl-2-oxo-3-oxabicyclo[3.1.0]hexane.

51. A process as claimed in Claim 50, in which the product is obtained enriched with the (1R,5S) isomer by using a catalyst bis{2 - butoxy - $\alpha$ - [2 - butoxy - 5 - (1,1 - dimethylethyl)phenyl] - 5 - (1,1 - dimethylethyl) - $\alpha$ - [(S) - 1 - ([(2 - hydroxyphenyl)methylene]amino)ethyl] - benzene methanolato(2 - )} - copper.

52. A process as claimed in Claim 50 or 51 characterized in that the 3-methyl-2-butenyl diazoacetate is prepared by treating 3-methyl-2-butenyl acetoacetate with an azide, followed by a base.

**Patentansprüche**

1. Eine Verbindung der Formel

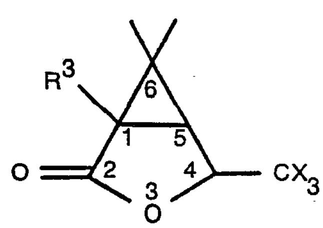

in der $R^3$ ein ($C_{1-6}$-Alkoxy)-carbonylrest und jedes X, das gleich oder verschieden ist, ein Chlor- oder Bromatom bedeutet.

2. 4-Trichlormethyl-6,6-dimethyl-2-oxo-3-oxabicyclo[3.1.0]hexan-1-carbonsäureäthylester.

3. 4-Trichlormethyl-6,6-dimethyl-2-oxo-3-oxabicyclo[3.1.0]hexan-1-carbonsäuremethylester.

4. Eine Verbindung der Formel

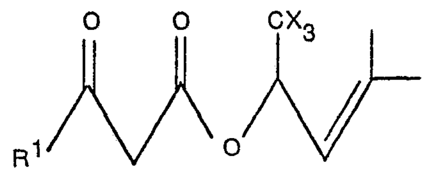

in der $R^1$ ein $C_{1-6}$-Alkylrest oder ein $C_{1-6}$-Alkoxyrest und jedes X, das gleich oder verschieden ist, ein Chlor- oder Bromatom bedeutet.

5. Eine Verbindung nach Anspruch 4, wobei $R^1$ ein $C_{1-6}$-Alkylrest ist.
6. Acetessigsäure-3-methyl-1-trichlormethyl-2-butenylester.
7. Acetessigsäure-(1R)-3-methyl-1-trichlormethyl-2-butenylester.
8. Acetessigsäure-3-methyl-1-tribrommethyl-2-butenylester.
9. Eine Verbindung nach Anspruch 4, wobei $R^1$ ein $C_{1-6}$-Alkoxyrest ist.
10. 3-Methyl-1-trichlormethyl-2-butenylmalonsäureäthylester.
11. Eine Verbindung der Formel

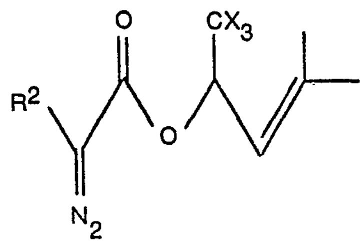

in der $R^2$ ein Wasserstoffatom, ein ($C_{1-6}$-Alkoxy)-carbonylrest oder ein $C_{1-6}$-Alkanoylrest und jedes X, das gleich oder verschieden ist, ein Chlor- oder Bromatom bedeutet.

12. Eine Verbindung nach Anspruch 11, wobei $R^2$ ein Wasserstoffatom ist.
13. Diazoessigsäure-3-methyl-1-trichlormethyl-2-butenylester.
14. Diazoessigsäure-(1R)-3-methyl-1-trichlormethyl-2-butenylester.
15. Diazoessigsäure-3-methyl-1-tribromethyl-2-butenylester.
16. Eine Verbindung nach Anspruch 11, wobei $R^2$ ein ($C_{1-6}$-Alkoxy)-carbonylrest ist.
17. Diazomalonsäureäthyl-3-methyl-1-trichlormethyl-2-butenylester.
18. Diazomalonsäuremethyl-3-methyl-1-trichlormethyl-2-butenylester.
19. Eine Verbindung nach Anspruch 11, wobei $R^2$ ein $C_{1-6}$-Alkanoylrest ist.
20. Acetodiazoessigsäure-1-trichlormethyl-3-methyl-2-butenylester.
21. Verfahren zur Herstellung eines Lactons der Formel

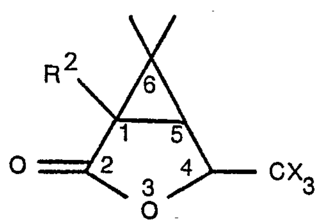

dadurch gekennzeichnet, daß man ein Diazoderivat der Formel

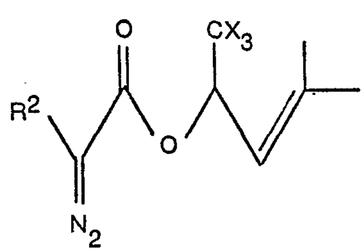

durch intramolekulare Carbenoid-Cyclisation cyclisiert, wobei jedes X ein Chlor- oder Bromatom und $R^2$ ein Wasserstoffatom oder ein ($C_{1-6}$-Alkoxy)-carbonylrest ist.

22. Verfahren nach Anspruch 21, dadurch gekennzeichnet, daß man das Diazoderivat mit einem kupferhaltigen Katalysator cyclisiert.

23. Verfahren nach Anspruch 22, dadurch gekennzeichnet, daß der Katalysator ein chiraler zweikerniger kupferhaltiger Katalysator ist.

24. Verfahren nach Anspruch 23, dadurch, gekennzeichnet, daß der Katalysator ein [N-(2-Hydroxyäthylat)-salicyliden-aminato]-kupfer(II)-zweikerniger Komplex ist.

25. Verfahren nach Anspruch 24, dadurch gekennzeichnet, daß der Katalysator Bis - {2 - butoxy - $\alpha$ - [2 - butoxy - 5 - (1,1 - dimethyläthyl) - phenyl] - 5 - (1,1 - dimethyläthyl) - $\alpha$ - [(S) - 1 - ([(2 - hydroxyphenyl) - methylen] - amino) - äthyl] - benzolmethanolato(2 - )} - kupfer ist.

26. Verfahren nach Anspruch 25, dadurch gekennzeichnet, daß mit dem (1R, 4R, 5S)-Isomer angereichertes 4-Trichlormethyl-6,6-dimethyl-2-oxo-3-oxabicyclo[3.1.0]hexan hergestellt wird aus Diazoessigsäure-3-methyl-1-trichlormethyl-2-butenylester.

27. Verfahren nach Anspruch 22, dadurch gekennzeichnet, daß das Diazoderivat und das Lacton optisch aktiv sind.

28. Verfahren nach Anspruch 27, dadurch gekennzeichnet, daß (1R, 4R, 5S)-4-Trichlormethyl-6,6-dimethyl-2-oxo-3-oxabicyclo[3.1.0]hexan praktisch frei von anderen Isomeren hergestellt wird aus Diazoessigsäure-(R)-3-methyl-1-trichlormethyl-2-butenylester.

29. Verfahren nach Anspruch 22, dadurch gekennzeichnet, daß 4-Trichlormethyl-6,6-dimethyl-2-oxo-3-oxabicyclo[3.1.0]hexan hergestellt wird aus Diazoessigsäure-3-methyl-1-trichlormethyl-2-butenylester.

30. Verfahren nach Anspruch 22, dadurch gekennzeichnet, daß 4-Tribrommethyl-6,6-dimethyl-2-oxo-3-oxabicyclo[3.1.0]hexan hergestellt wird aus Diazoessigsäure-3-methyl-1-tribrommethyl-2-butenylester.

31. Verfahren nach Anspruch 22, dadurch gekennzeichnet, daß 4-Trichlormethyl-6,6-dimethyl-2-oxo-3-oxabicyclo[3.1.0]hexan-1-carbonsäureäthylester hergestellt wird aus Diazomalonsäureäthyl-3-methyl-1-trichlormethyl-2-butenylester.

32. Verfahren nach Anspruch 22, dadurch gekennzeichnet, daß der 4-Trichlormethyl-6,6-dimethyl-2-oxo-3-oxabicyclo[3.1.0]hexan-1-carbonsäuremethylester hergestellt wird aus Diazomalonsäuremethyl-3-methyl-1-trichlormethyl-2-butenylester.

33. Verfahren nach einem der Ansprüche 21 bis 32, dadurch gekennzeichnet, daß das hergestellte Lacton mit einem Metall ausgewählt von Zink, Magnesium, Natrium und Koordinationskomplexen von Cr(II) in einem Lösungsmittel ausgewählt von Essigsäure, Alkoholen und Äthern und deren Gemischen behandelt wird unter Bildung einer Verbindung der Formel

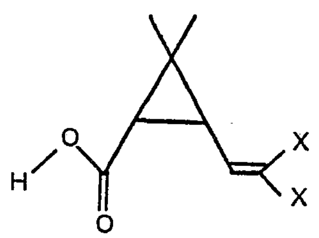

in der die Carboxylgruppe und die Dihalogenvinylgruppe zueinander in cis-Stellung stehen.

34. Verfahren nach Anspruch 33, dadurch gekennzeichnet, daß cis-3-(2,2,-Dichlorvinyl)-2,2-dimethylcyclopropancarbonsäure hergestellt wird aus 4-Trichlormethyl-6,6-dimethyl-2-oxo-3-oxabicyclo[3.1.0]hexan.

35. Verfahren nach Anspruch 33, dadurch gekennzeichnet, daß (1R, 3R)-3-(2,2-Dichlorvinyl)-2,2-dimethylcyclopropancarbonsäure hergestellt wird aus (1R, 4R, 5S)-6,6-Dimethyl-2-oxo-4-trichlormethyl-3-oxabicyclo[3.1.0]hexan.

36. Verfahren nach Anspruch 33, dadurch gekennzeichnet, daß cis-3-(2,2-Dibromvinyl)-2,2-dimethylcyclopropancarbonsäure hergestellt wird aus 4-Tribrommethyl-6,6-dimethyl-2-oxo-3-oxabicyclo[3.1.0]hexan.

37. Verfahren nach einem der Ansprüche 21 bis 25 und 27, wobei $R^2$ ein ($C_{1-6}$-Alkoxy)-carbonylrest ist, dadurch gekennzeichnet, daß das erhaltene Lacton dealkoxycarbonyliert wird zum entsprechenden Lacton, in dem $R^2$ ein Wasserstoffatom bedeutet.

38. Verfahren nach Anspruch 37, dadurch gekennzeichnet, daß die Dealkoxycarbonylierung durchgeführt wird mit Lithiumchlorid in einem polaren Lösungsmittel.

39. Verfahren nach Anspruch 38, dadurch gekennzeichnet, daß in der Dealkoxycarbonylierungsstufe das 4-Trichlormethyl-6,6-dimethyl-2-oxobicyclo[3.1.0]hexan hergestellt wird aus dem 4-Trichlormethyl-6,6-dimethyl-2-oxo-3-oxabicyclo[3.1.0]hexan-1-carbonsäuremethyl- oder -äthylester.

40. Verfahren nach Anspruch 21, dadurch gekennzeichnet, daß das Diazoderivat der Formel

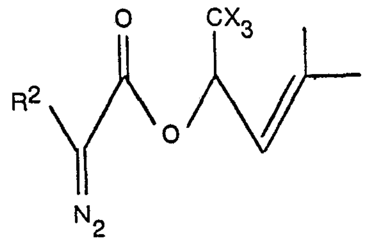

hergestellt wird aus einem Ester der Formel

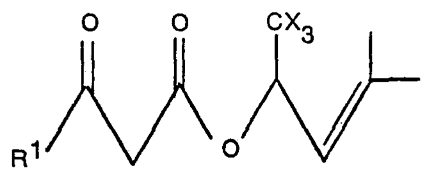

in der jedes X ein Chlor- oder Bromatom, $R^1$ ein $C_{1-6}$-Alkyl- oder $C_{1-6}$-Alkoxyrest und $R^2$ ein Wasserstoffatom oder ein ($C_{1-6}$-Alkoxy)-carbonylrest bedeutet, durch Umsetzung des Esters mit einem Azid.

41. Verfahren nach Anspruch 40, dadurch gekennzeichnet, daß ein Diazoderivat, in dem $R^2$ ein Wasserstoffatom bedeutet, hergestellt wird durch Behandlung eines Esters, in dem $R^1$ einen $C_{1-6}$-Alkylrest bedeutet, mit einem Azid und anschließend mit einer Base.

42. Verfahren nach Anspruch 41, dadurch gekennzeichnet, daß Diazoessigsäure-3-methyl-1-trichlormethyl-2-butenylester hergestellt wird aus Acetessigsäure-3-methyl-1-trichlormethyl-2-butenylester.

43. Verfahren nach Anspruch 41, dadurch gekennzeichnet, daß Diazoessigsäure-3-methyl-1-tribrommethyl-2-butenylester hergestellt wird aus Acetessigsäure-3-methyl-1-tribrommethyl-2-butenylester.

44. Verfahren nach Anspruch 41, dadurch gekennzeichnet, daß Diazoessigsäure-(1R)-3-methyl-1-trichlormethyl-2-butenylester hergestellt wird aus Acetessigsäure-(1R)-3-methyl-1-trichlormethyl-2-butenylester.

45. Verfahren nach Anspruch 40, dadurch gekennzeichnet, daß ein Diazoderivat, in dem $R^2$ ein ($C_{1-6}$-Alkoxy)-carbonylrest ist, hergestellt wird durch Umsetzung eines Esters, in dem $R^1$ ein $C_{1-6}$-Alkoxyrest ist, mit einem Azid.

46. Verfahren nach Anspruch 45, dadurch gekennzeichnet, daß Diazomalonsäureäthyl-3-methyl-1-trichlormethyl-2-butenylester hergestellt wird aus Malonsäureäthyl-3-methyl-1-trichlormethyl-2-butenylester.

47. Verfahren nach Anspruch 40, dadurch gekennzeichnet, daß ein Diazoester der Formel

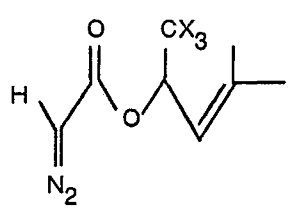

hergestellt wird durch Umsetzung eines Alkohols der Formel

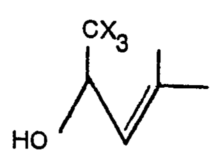

mit einem Hydrazon von Glyoxylylchlorid, wobei X die in Anspruch 1 angegebene Bedeutung hat.

48. Verfahren nach Anspruch 47, dadurch gekennzeichnet, daß Diazoessigsäure-3-methyl-1-trichlormethyl-2-butenylester hergestellt wird durch Umsetzung von 1,1,1-Trichlor-4-methyl-3-penten-2-ol mit dem p-Toluolsulfonylhydrazon von Glyoxyloylchlorid.

49. Diazoessigsäure-3-methyl-2-butenylester.

50. Das Verfahren, bei dem Diazoessigsäure-3-methyl-2-butenylester durch intramolekulare

Carbenoidcyclisation zum 6,6-Dimethyl-2-oxo-3-oxabicyclo[3.1.0]hexan cyclisiert wird.

51. Verfahren nach Anspruch 50, bei dem das Produkt mit dem (1R,5S)-Isomer angereichert erhalten wird unter Verwendung von Bis - {2 - butoxy - $\alpha$ - [2 - butoxy - 5 - (1,1 - dimethyl - äthyl) - phenyl] - 5 - (1,1 - dimethyläthyl) - $\alpha$ [(S) - 1 - ([(2 - hydroxyphenyl) - methylen] - amino) - äthyl] - benzol - methanolato(2 - )} - Kupfer als Katalysator.

52. Verfahren nach Anspruch 50 oder 51, dadurch gekennzeichnet, daß der Diazoessigsäure-3-methyl-2-butenylester hergestellt wird durch Behandlung von Acetessigsäure-3-methyl-2-butenylester mit einem Azid und anschließend mit einer Base.

## Revendications

1. Composé répondant à la formule:

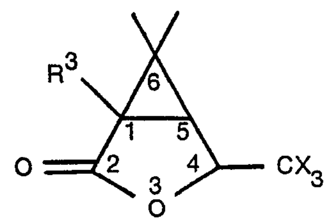

dans laquelle $R^3$ est un alkoxy en $C_{1-6}$ et chacun des X, qui sont identiques ou différents est un atome de chlore ou de brome.

2. 4-trichlorométhyl-6,6-diméthyl-2-oxo-3-oxabicyclo[3.1.0]hexane-1-carboxylate d'éthyle.

3. 4-trichlorométhyl-6,6-diméthyl-2-oxo-3-oxabicyclo[3.1.0]hexane-1-carboxylate de méthyle.

4. Composé répondant à la formule:

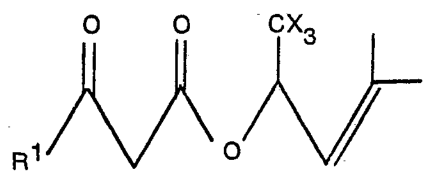

dans laquelle $R^1$ est un alkyle en $C_1$ à $C_6$ ou un alkoxy en $C_1$ à $C_6$ et chacun des X, qui sont identiques ou différents, est un atome de chlore ou de brome.

5. Composé selon la revendication 4, caractérisé en ce que $R^1$ est un alkyle en $C_1$ à $C_6$.

6. Acétoacétate de 3-méthyl-1-trichlorométhyl-2-butényle.

7. Acétoacétate de (1R)-3-méthyl-1-trichlorométhyl-2-butényle.

8. Acétoacétate de 3-méthyl-1-tribromométhyl-2-butényle.

9. Composé selon la revendication 4, caractérisé en ce que $R^1$ est un alkoxy en $C_1$ à $C_6$.

10. 3-méthyl-1-trichlorométhyl-2-buténylе-malonate-d'éthyle.

11. Composé répondant à la formule:

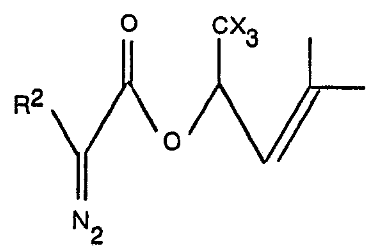

dans laquelle $R^2$ est l'hydrogène, un (alkoxy en $C_1$ à $C_6$) carbonyle ou un alkanoyle en $C_{1-6}$ et chacun des X, qui sont identiques ou différents, est un atome de chlore ou de brome.

12. Composé selon la revendication 11, caractérisé en ce que $R^2$ est l'hydrogène.

13. Diazoacétate de 3-méthyl-1-trichlorométhyl-2-butényle.

14. Diazoacétate de (1R)-3-méthyl-1-trichlorométhyl-2-butényle.

15. Diazoacétate de 3-méthyl-1-tribromométhyl-2-butényle.

16. Composé selon la revendication 11, caractérisé en ce que $R^2$ est un (alkoxy en $C_{1-6}$) carbonyle.

17. 3-méthyl-1-trichlorométhyl-2-buténуl-diazomalonate d'éthyle.

18. 3-méthyl-1-trichlorométhyl-2-buténуl-diazomalonate de méthyle.

19. Composé selon la revendication 11, caractérisé en ce que $R^2$ est un alkanoyle en $C_{1-6}$.

20. Acétodiazoacétate de 1-trichlorométhyl-3-méthyl-2-butényle.

21. Procédé de préparation d'une lactone de formule:

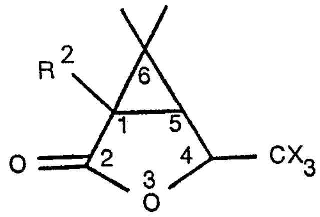

caractérisé en ce que l'on cyclise un dérivé diazoïque de formule:

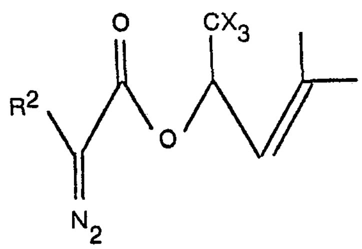

par cyclisation intramoléculaire du type carbénoïde, où chacun des X est un atome de chlore ou de brome et $R^2$ est l'hydrogène ou un alkoxy en $C_{1-6}$ carbonyle.

22. Procédé selon la revendication 21, caractérisé en ce que l'on cyclise le dérivé diazoïque avec un catalyseur contenant du cuivre.

23. Procédé selon la revendication 22, caractérisé en ce que le catalyseur est un catalyseur contenant du cuivre binucléaire à pouvoir rotatoire.

24. Procédé selon la revendication 23, caractérisé en ce que le catalyseur est un complexe binucléaire de [N-(2-hydroxyéthylate)salicylidène aminato]-cuivre (II).

25. Procédé selon la revendication 24, caractérisé en ce que le catalyseur est le bis{2 - butoxy - $\alpha$ - [2 - butoxy - 5 - (1,1 - diméthyléthyl) - phényl] - 5 - (1,1 - diméthyléthyl) - $\alpha$ - [(S) - 1 - ([(2 - hydroxyphényl)méthylène] - amino)éthyl] benzène méthanolato(2-)}-cuivre.

26. Procédé selon la revendication 25, caractérisé en ce que l'on prépare du 4-trichlorométhyl-6,6-diméthyl-2-oxo-3-oxabicyclo(3.1.0)hexane enrichi en isomère (1R, 5S) à partir de diazoacétate de 3-méthyl-1-trichlorométhyl-2-butényle.

27. Procédé selon la revendication 22, caractérisé en ce que le dérivé diazoïque et la lactone sont optiquement actifs.

28. Procédé selon la revendication 27, caractérisé en en ce qu'on prépare du (1R,4R,5S)-4-trichlorométhyl-6,6-diméthyl-2-oxo-3-oxabicyclo-(3.1.0)hexane pratiquement exempt d'autres isomères de celui-ci, à partir du diazoacétate de (R)-3-méthyl-1-trichlorométhyl-2-butényle.

29. Procédé selon la revendication 22, caractérisé en ce que l'on prépare du 4-trichlorométhyl-6,6-diméthyl-2-oxo-3-oxabicyclo(3.1.0)hexane à partir du diazoacétate de 3-méthyl-1-trichlorométhyl-2-butényle.

30. Procédé selon la revendication 22, caractérisé en ce que l'on prépare du 4-tribromométhyl-6,6-diméthyl-2-oxo-3-oxabicyclo(3.1.0)hexane à partir du diazoacétate de 3-méthyl-1-tribromométhyl-2-butényle.

31. Procédé selon la revendication 22, caractérisé en ce que l'on prépare du 4-trichlorométhyl-6,6-diméthyl-2-oxo-3-oxabicyclo(3.1.0)hexane-1-carboxylate d'éthyle à partir du 3-méthyl-1-trichlorométhyl-2-buténylе-diazomalonate d'éthyle.

32. Procédé selon la revendication 22, caractérisé en ce qu'on prépare du 4-trichlorométhyl-6,6-diméthyl-2-oxo-3-oxabicyclo(3.1.0)hexane-1-carboxylate de méthyle à partir du 3-méthyl-1-trichlorométhyl-2-buténыl-diazomalonate de méthyle.

33. Procédé selon l'une quelconque des revendications 21 à 32, caractérisé en ce que l'on traite la lactone ainsi préparée par un métal choisi parmi le zinc, le magnésium, le sodium et des complexes de coordination du Cr(II), dans un solvant choisi parmi l'acide acétique, des alcools et des éthers, et des mélanges de ceux-ci, pour produire un composé de formule:

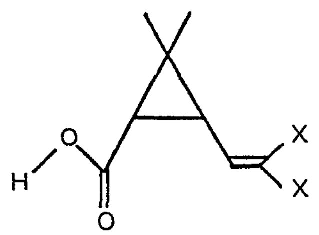

dans laquelle les groupes carboxy et dihalovinyle sont *cis* l'un par rapport à l'autre.

34. Procédé selon la revendication 33, caractérisé en ce que l'on prépare de l'acide *cis*-3-(2,2-dichlorovinyl)-2,2-diméthylcyclopropanecarboxylique à partir du 4-trichlorométhyl-6,6-diméthyl-2-oxo-2-oxabicyclo(3.1.0)hexane.

35. Procédé selon la revendication 33, caractérisé en ce que l'on prépare l'acide (1R,3R)-3-(2,2-dichlorovinyl)-2,2-diméthylcyclopropanecarboxylique à partir du (1R,4R,5S) - 6,6 - diméthyl - 2 - oxo - 4 - trichlorométhyl - 3 - oxabicyclo(3.1.0)hexane.

36. Procédé selon la revendication 33, caractérisé en ce qu'on prépare de l'acide *cis*-3-(2,2-dibromovinyl)-2,2 - diméthylcyclopropanecarboxylique à partir du 4-tribromométhyl - 6,6 - diméthyl - 2 - oxo - 3 - oxabicyclo(3.1.0)hexane.

37. Procédé selon l'une quelconque des revendications 21 à 25 et 27, dans lesquelles $R^2$ est un (alkoxy en $C_{1-6}$) carboxyle, caractérisé en ce que la lactone obtenue est désalkoxycarbonylée pour donner la lactone correspondante dans laquelle $R^2$ est l'hydrogène.

38. Procédé selon la revendication 37, caractérisé en ce que la désalkoxycarbonylation s'effectue avec du chlorure de lithium dans un solvant polaire.

39. Procédé selon la revendication 38, caractérisé en ce que, au cours de l'opération de désalkoxycarbonylation, on prépare du 4-trichlorométhyl - 6,6 - diméthyl - 2 - oxobicyclo(3.1.0]-hexane à partir du 4 - trichloro - méthyl - 6,6 - diméthyl - 2 - oxo - 3 - oxabicyclo(3.1.0)-hexane - 1 - carboxylate de méthyle ou d'éthyle.

40. Procédé selon la revendication 21, caractérisé en ce que le dérivé diazoïque de formule:

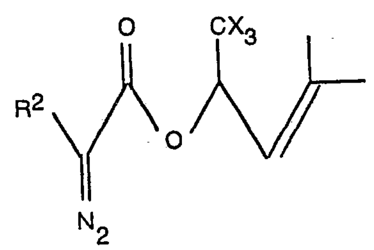

est préparé à partir d'un ester de formule:

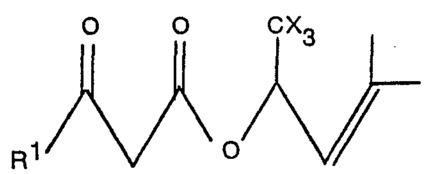

dans laquelle chacun des X est un atome de chlore ou de brome, $R^1$ est un alkyle en $C_{1-6}$ ou un alkoxy en $C_{1-6}$ et $R^2$ est l'hydrogène ou un (alkoxy en $C_{1-6}$) carbonyle, par réaction de l'éther avec un azothydrure.

41. Procédé selon la revendication 40, caractérisé en ce que l'on prépare un dérivé diazoïque dans lequel $R^2$ est l'hydrogène par traitement d'un ester dans lequel $R^1$ est un alkyle en $C_{1-6}$ par un azothydrure et par une base.

42. Procédé selon la revendication 41, caractérisé en ce que l'on prépare du diazoacétate de 3-méthyl-1-trichlorométhyl-2-buténylе à partir de l'acétoacétate de 3 - méthyl - 1 - trichlorométhyl - 2 - buténylе.

43. Procédé selon la revendication 41, caractérisé en ce que l'on prépare du diazoacétate de 3-méthyl-1-tribromométhyl-2-buténylе à partir de l'acétoacétate de 3-méthyl-1-tribromométhyl-2-buténylе.

44. Procédé selon la revendication 41, caractérisé en ce que l'on prépare du diazoacétate de (IR)-3-méthyl-1-trichlorométhyl-2-buténylе à partir de l'acétoacétate de (IR)-3-méthyl-1-trichlorométhyl-2-buténylе.

45. Procédé selon la revendication 40, caractérisé en ce que l'on prépare un dérivé diazoïque dans

lequel $R^2$ est un (alkoxy en $C_{1-6}$)carbonyle par traitement par un azothydrure et un ester dans lequel $R^1$ est un alkoxy en $C_{1-6}$.

46. Procédé selon la revendication 45, caractérisé en ce que l'on prépare du 3-méthyl-1-trichlorométhyl-2-butènyl-diazomalonate d'éthyle à partir de 3-méthyl-1-trichlorométhyl-2-butènyl-malonate d'éthyle.

47. Procédé suivant la revendication 40, caractérisé en ce que l'on prépare un ester diazoïque de formule:

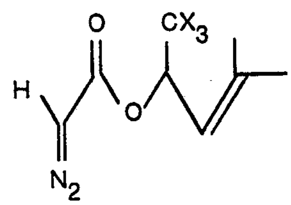

par réaction d'un alcool de formule:

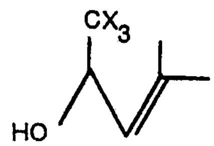

avec une hydrazone de chlorure de glyoxyloyle, X étant tel que défini dans la revendication 1.

48. Procédé suivant la revendication 47, caractérisé en ce que l'on prépare du diazoacétate de 3-méthyl-1-trichlorométhyl-2-butényle par réaction de 1,1,1-trichloro-4-méthyl-3-pentèn-2-ol avec de la p-toluène sulfonyle hydrazone de chlorure de glyoxyloyle.

49. Diazoacétate de 3-méthyl-2-butényle.

50. Procédé caractérisé en ce que l'on cyclise du diazoacétate de 3-méthyl-2-butényle par cyclisation intramoléculaire de type carbénoïde pour produire du 6,6-diméthyl-2-oxo-3-oxabicyclo-(3.1.0)hexane.

51. Procédé suivant la revendication 50, caractérisé en ce que le produit est obtenu enrichi en isomère (1R,5S) par utilisation comme catalyseur de bis{2 - butoxy - $\alpha$ - [2 - butoxy - 5 - (1,1 - diméthyléthyl)phényl] - 5 - (1,1 - diméthyléthyl) - $\alpha$ - [(S) - 1 - ([(2 - hydroxyphényl)méthylène]amino)éthyl] - benzène méthanolato (2-)} - cuivre.

52. Procédé suivant la revendication 50 ou 51, caractérisé en ce que l'on prépare le diazoacétate de 3-méthyl-2-butényle par traitement d'acétoacétate de 3-méthyl-2-butényle par un azothydrure puis par une base.